# FASCICULE DE BREVET EUROPEEN

(11) **EP 3 094 645 B1**
(45) Date de publication et mention de la délivrance du brevet: **24.07.2019**
(21) Numéro de dépôt: 15704054.4
(22) Date de dépôt: 16.01.2015
(51) Int. Cl.: C07K 16/12, C12N 15/13, A61K 39/395, A61P 31/04, A61K 47/42, G01N 33/569, C12N 15/63, A61K 39/40, G01N 33/53

(54) **IMMUNOGLOBULINE ANTI-TOXINE DU CHARBON**
IMMUNGLOBULIN GEGEN DAS ANTHRAX-TOXIN
IMMUNOGLOBULIN AGAINST THE ANTHRAX TOXIN

(30) Priorité: 17.01.2014 FR 1450405
(43) Date de publication de la demande: 23.11.2016
(73) Titulaire: Laboratoire Français du Fractionnement et des Biotechnologies, 91940 Les Ulis (FR); Etat Français représenté par le Directeur Central du Service de Santé des Armées, 75509 Paris Cedex 15 (FR)
(72) Inventeur: BEHRENS, Christian, F-91430 Vauhallan (FR); KLEIN, Philippe, F-30100 Alès (FR); HOGUET, Denis, F-30560 Saint Hilaire de Brethmas (FR)
(74) Mandataire: Cabinet Chaillot
(86) Numéro de dépôt international: PCT/FR2015/050113
(87) Numéro de publication internationale: WO 2015/107307

(56) Documents cités:
- WO-A-2007/084107
- WO-A-2009/050388
- WO-A2-2009/071860
- US-A1- 2006 121 045
- LAFFLY EMMANUELLE ET AL: "Selection of a macaque Fab with framework regions like those in humans, high affinity, and ability to neutralize the protective antigen (PA) of Bacillus anthracis by binding to the segment of PA between residues 686 and 694", ANTIMICROBIAL AGENTS AND CHEMOTHERAPY, AMERICAN SOCIETY FOR MICROBIOLOGY, WASHINGTON, DC, US, vol. 49, no. 8, août 2005 (2005-08), pages 3414-3420, XP002444247, ISSN: 0066-4804
- LAFFLY E ET AL: "Improvement of an Antibody Neutralizing the Anthrax Toxin by Simultaneous Mutagenesis of Its Six Hypervariable Loops", JOURNAL OF MOLECULAR BIOLOGY, LONDON, GB, vol. 378, no. 5, 16 mai 2008 (2008-05-16), pages 1094-1103, XP022627585, ISSN: 0022-2836 [extrait le 2008-03-28]
- PRESTA ET AL: "Engineering of therapeutic antibodies to minimize immunogenicity and optimize function", ADVANCED DRUG DELIVERY REVIEWS, AMSTERDAM, NL, vol. 58, no. 5-6, 7 août 2006 (2006-08-07) , pages 640-656, XP005611419, ISSN: 0169-409X
- WARK ET AL: "Latest technologies for the enhancement of antibody affinity", ADVANCED DRUG DELIVERY REVIEWS, AMSTERDAM, NL, vol. 58, no. 5-6, 7 août 2006 (2006-08-07) , pages 657-670, XP005611420, ISSN: 0169-409X

## Description

La présente invention se rapporte à une immunoglobuline dirigée contre l'antigène protecteur de la toxine du charbon.

Le charbon est une maladie infectieuse causée par une bactérie à gram positif, *Bacillus anthracis.* Cette bactérie est non mobile, et forme des spores, hautement résistantes, qui germinent en une forme végétative lorsqu'elles se trouvent dans des environnements tels que le sang ou les tissus des hommes ou des animaux. Malgré leur haute résistance, les spores ne se reproduisent pas, en revanche elles peuvent survivre des dizaines d'années dans le sol.

Une infection par le charbon peut prendre trois formes : cutanée, pulmonaire ou digestive. L'infection pulmonaire est la plus fréquemment mortelle. En cas d'inhalation, les spores de *B. anthracis* passent dans les alvéoles où elles sont phagocytées par les macrophages et les cellules dendritiques, notamment. Les spores germent dans ces cellules et les formes végétatives se multiplient dans les ganglions. Les bactéries passent alors dans le sang, se reproduisent en continu et produisent des toxines, responsables en partie de la létalité de la maladie.

Les toxines du charbon sont composées de trois protéines distinctes : l'antigène protecteur (PA, 83 kDa avant clivage enzymatique et 63 kDa après clivage), le facteur létal (LF, 90 kDa) et le facteur oedémateux (EF, 89 kDa). La toxine létale, qui joue un rôle prépondérant dans la pathogénicité, est formée de PA et LF ; et la toxine oedémateuse, dont le rôle dans la physiologie de la maladie est moindre, de PA et EF. Ces protéines sont sécrétées par la bactérie en tant que monomères non toxiques, et s'assemblent à la surface des cellules cibles pour former des complexes toxiques.

Jusqu'à présent, plusieurs antibiotiques, tels que la pénicilline, la doxycycline et les fluoroquinolones (par exemple la ciprofloxacine), ont été utilisés pour le traitement des infections par le charbon. Cependant, certains de ces antibiotiques peuvent ne pas avoir d'effets sur certaines souches de *B. anthracis,* résistantes aux antibiotiques. De plus, les antibiotiques n'ayant pas d'action inhibitrice vis-à-vis des toxines du charbon, ils doivent être nécessairement administrés à des stades très précoces de l'infection ; or les diagnostics précoces sont difficiles à établir car les symptômes initiaux sont non spécifiques.

Des vaccins, dont le composant majeur est l'antigène protecteur PA, ont été développés mais ne sont utilisés que pour des personnes susceptibles d'être en contact avec *B*. *anthracis.* De plus, du fait de la nécessité d'une période de plusieurs mois pour acquérir une immunité suffisante, ces vaccins ne peuvent pas être utilisés dans des situations d'urgence. En France actuellement, aucun de ces vaccins n'est agréé pour l'utilisation humaine. Il est donc nécessaire de développer de nouvelles approches thérapeutiques et préventives, autres que les antibiotiques.

L'immunisation passive avec des anticorps représente une stratégie efficace pour neutraliser la toxine. Plusieurs essais ont été réalisés pour neutraliser la toxine létale du charbon à l'aide d'anticorps monoclonaux contre l'antigène protecteur (PA) et le facteur létal (LF). La neutralisation de la toxine létale du charbon à l'aide d'un anticorps peut avoir lieu par inhibition de la liaison de PA à son récepteur cellulaire, inhibition du clivage de PA, inhibition de la liaison entre PA et LF ou encore inhibition de l'action de LF par exemple.
Le développement de nouveaux anticorps permettant de neutraliser la toxine du charbon est ainsi d'un intérêt général pour une prévention et un traitement efficace du charbon.

Dans un travail récent, un macaque a été immunisé avec l'antigène protecteur PA83 pour obtenir des anticorps destinés à traiter l'infection humaine par le charbon. A partir de la moelle osseuse, les gènes codant des fragments d'anticorps Fab reconnaissant spécifiquement PA83 ont été amplifiés et clonés pour obtenir une librairie de fragments.

Un fragment de haute affinité (Kd=3,4 nM) et neutralisant efficacement la toxine létale (concentration d'inhibition à 50% = 5,6 +/- 0,13 nM), désigné sous le nom de 35PA83, a ensuite été isolé (Laffly et al., antimicrobial agents and chemotherapy, 2005, 49(8) : 3414-3420 et Laffly et al., Journal of molecular biology, 2008, 378(5) : 1094-1103). Le fragment d'immunoglobuline 35PA83 neutralise la toxine du charbon en empêchant l'interaction de PA avec son récepteur cellulaire.

Une immunoglobuline chimérique de 35PA83 appelée «v2», dont les régions variables sont dérivées du fragment 35PA83, a été préparée et décrite dans la demande internationale WO 2009/071860 et WO 2009/050388.

Néanmoins, l'augmentation de l'affinité de ce fragment d'immunoglobuline vis-à-vis de l'antigène PA présenterait le double avantage de réduire la quantité d'immunoglobuline à administrer au patient et de réduire le coût du traitement, mais également celui de permettre une meilleure détection de la toxine du charbon.

Un des buts de l'invention est ainsi de fournir une immunoglobuline présentant une haute affinité vis-à-vis de l'antigène PA de la toxine du charbon, et permettant une détection très sensible de la toxine du charbon.
Un autre but de l'invention est de fournir une immunoglobuline permettant le traitement ou la prévention de pathologies lies à la toxine du charbon.
Un autre but de l'invention est de fournir une méthode de détection de la toxine du charbon.
Encore un autre but de l'invention est également de fournir un kit de détection de la toxine du charbon.

La présente invention a ainsi pour objet une immunoglobuline de classe G dirigée contre l'antigène protecteur de la toxine du charbon, dans laquelle :
- chacune des chaînes lourdes comprend, ou consiste en, une séquence d'acides aminés représentée par SEQ ID NO : 5, et
- chacune des chaînes légères comprend, ou consiste en, une séquence d'acides aminés représentée par SEQ ID NO : 6.

La présente demande décrit une immunoglobuline de classe G dirigée contre l'antigène protecteur de la toxine du charbon, ou un de ses fragments, comprenant au moins :
- une région variable de chaîne lourde comprenant une séquence d'acides aminés possédant au moins 90% d'identité avec la séquence SEQ ID NO : 1, étant entendu qu'elle comprend les acides aminés Leucine en position 51 et Glycine en position 67, et/ou
- une région variable de chaîne légère comprenant une séquence d'acides aminés possédant au moins 90% d'identité avec la séquence SEQ ID NO : 2, étant entendu qu'elle comprend l'acide aminé Leucine en position 55.

La séquence SEQ ID NO : 1 comprend une leucine en position 51 et une glycine en position 67. La séquence SEQ ID NO : 2 comprend une leucine en position 55. Les séquences selon l'invention présentent au moins 90% d'identité avec SEQ ID NO : 1 ou 2, mais contiennent nécessairement, selon les cas, la leucine en position 51 et la glycine en position 67 (SEQ ID NO : 1), ou la leucine en position 55 (SEQ ID NO : 2).

De préférence, l'immunoglobuline ou un de ses fragments selon l'invention a une affinité pour l'antigène protecteur de la toxine du charbon inférieure à 2.10⁻⁹ M, de préférence inférieure à 1.10⁻⁹ M, de préférence inférieure ou égale à 3,3.10⁻¹¹ M.

Le terme *"immunoglobuline"* est équivalent au terme *"anticorps",* c'est-à-dire qu'il fait référence à une protéine multimérique constituée de 4 chaînes participant à la réponse immunitaire acquise.

Par «*fragment d'immunoglobuline*», on entend un fragment ayant la capacité de se lier à l'antigène PA. De préférence, un tel fragment comprend au moins :
- une région variable de chaîne lourde comprenant une séquence d'acides aminés représentée par la séquence SEQ ID NO : 1, et/ou
- une région variable de chaîne légère comprenant une séquence d'acides aminés représentée par la séquence SEQ ID NO : 2.

Ainsi, d'une part, on peut utiliser une immunoglobuline de l'invention, i.e. une molécule d'immunoglobuline entière, c'est-à-dire une immunoglobuline constituée de deux chaînes lourdes et de deux chaînes légères complètes. D'autre part, un fragment d'immunoglobuline selon l'invention peut être utilisé. Des fragments d'immunoglobuline bien connus sont par exemple les fragments F(ab')2, Fab, Fv, scFv et Fd.

Les immunoglobulines de type G (IgG) sont des hétérodimères constitués de 2 chaînes lourdes et de 2 chaînes légères, liées entre elles par des ponts disulfures. Chaque chaîne est constituée, en position N-terminale, d'une région ou domaine variable (codée par les gènes réarrangés V-J pour la chaîne légère et V-D-J pour la chaîne lourde) spécifique de l'antigène contre lequel l'immunoglobuline est dirigée, et en position C-terminale, d'une région constante, constituée d'un seul domaine CL pour la chaîne légère ou de 3 domaines (CH1, CH2 et CH3) pour la chaîne lourde.

L'association des domaines variables et des domaines CH1 et CL des chaînes lourdes et légères forme les parties Fab, qui sont connectées à la région Fc par une région charnière très flexible permettant à chaque Fab de se fixer à sa cible antigénique, tandis que la région Fc, médiatrice des propriétés effectrices de l'anticorps, reste accessible aux effecteurs immunitaires, phagocytes ou cellules tueuses, et le complément ; ces régions constantes ne sont pas impliquées dans la liaison à l'antigène.

La région Fc, constituée des 2 domaines globulaires CH2 et CH3, est glycosylée au niveau du domaine CH2 avec la présence, sur chacune des 2 chaînes, d'un N-glycanne biantenné, lié à l'asparagine en position 297 de la séquence de chaine lourde des anticorps selon la numérotation EU numbering (Asn 297 - cf Edelman, G.M. et al., Proc. Natl. Acad. USA, 63, 78-85 (1969), et site IMGT http://www.imgt.org/IMGTScientificChart/Numbering/Hu_IGHGnber.html).
Dans la présente demande, par « Asn 297 », on entend en réalité l'asparagine en position 305 de la séquence SEQ ID NO :5.

La région variable est, quant à elle, impliquée dans la liaison de l'immunoglobuline à son épitope.

Une immunoglobuline dont la région constante (Fc) a été enzymatiquement clivée, de façon à en préserver la région charnière, est désignée comme un fragment F(ab')2 et conserve les deux sites de liaison à l'antigène.
De même, une immunoglobuline dont la région constante, y compris la région charnière a été enzymatiquement clivée, ou qui a été produite sans cette région, est désignée comme un fragment Fab et conserve un des deux sites de liaison à l'antigène.

Le fragment Fd est formé des régions VH et CH1.

Dans la région variable, on trouve les régions déterminant la complémentarité (CDRs, *complementary determining regions*), aussi appelées régions hypervariables, qui interagissent directement avec l'antigène et ont donc un impact déterminant pour l'affinité d'un anticorps pour sa cible antigénique.

Dans la région variable, on trouve un second type de régions, appelées régions charpentes (FRs, *frameworks*), qui maintiennent la structure tertiaire des CDRs. Ces régions charpentes sont relativement spécifiques de l'espèce où a été produite l'immunoglobuline. Dans le fragment Fd de la chaîne lourde et dans la chaîne légère, on trouve quatre régions charpentes (FR1 à 4) séparées respectivement par trois CDR (CDR1 à 3).

Selon l'invention, une séquence possédant au moins 90% d'identité avec une séquence de référence, possède au moins 90 %, de préférence au moins 91%, 92%, 93%, 94%, 95%, 96%, 97%, 98% ou 99%, d'identité en acides aminés avec ladite séquence de référence.

Le *"pourcentage d'identité"* entre deux séquences, au sens de la présente invention, est déterminé en comparant les deux séquences alignées de manière optimale, à travers une fenêtre de comparaison. La partie de la séquence d'acides aminés dans la fenêtre de comparaison peut ainsi comprendre des additions ou des délétions (par exemple des "gaps") par rapport à la séquence de référence (qui ne comprendrait pas ces additions ou ces délétions) de manière à obtenir un alignement optimal entre les deux séquences.
Le pourcentage d'identité est calculé en déterminant le nombre de positions pour lesquelles un résidu d'acide aminé est identique chez les deux séquences comparées, puis en divisant le nombre de positions pour lesquelles il y a identité entre les deux résidus d'acides aminés, par le nombre total de positions dans la fenêtre de comparaison, puis en multipliant le résultat par cent afin d'obtenir le pourcentage d'identité en acides aminés des deux séquences entre elles.

L'invention repose sur l'observation surprenante faite par les inventeurs que la combinaison d'une double mutation spécifique de la chaîne lourde (qui aboutit à une leucine en position 51 et à une glycine en position 67 dans SEQ ID NO :1) et une mutation spécifique de la chaîne légère (qui aboutit à une leucine en position 55 dans SEQ ID NO :2) entraîne une meilleure affinité de l'immunoglobuline 35PA83 vis-à-vis de la toxine du charbon par rapport aux immunoglobulines ou fragments d'immunoglobulines de l'art antérieur.

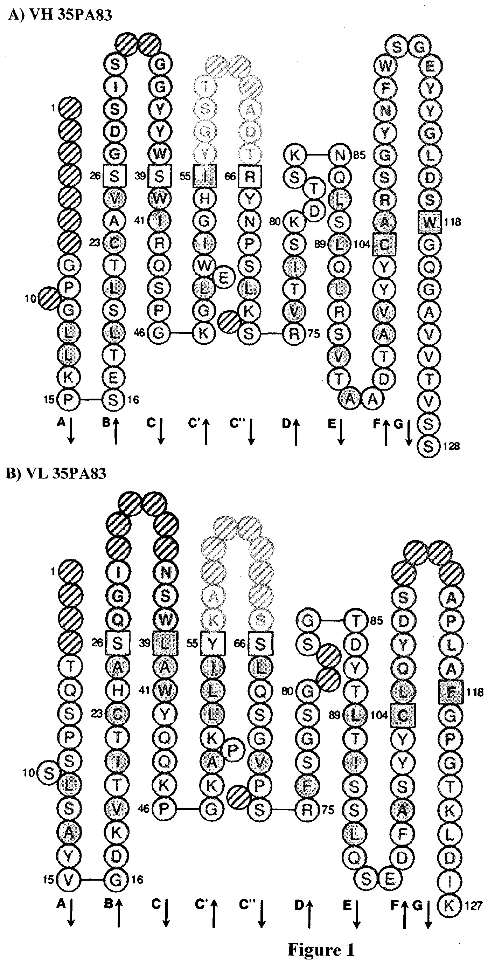

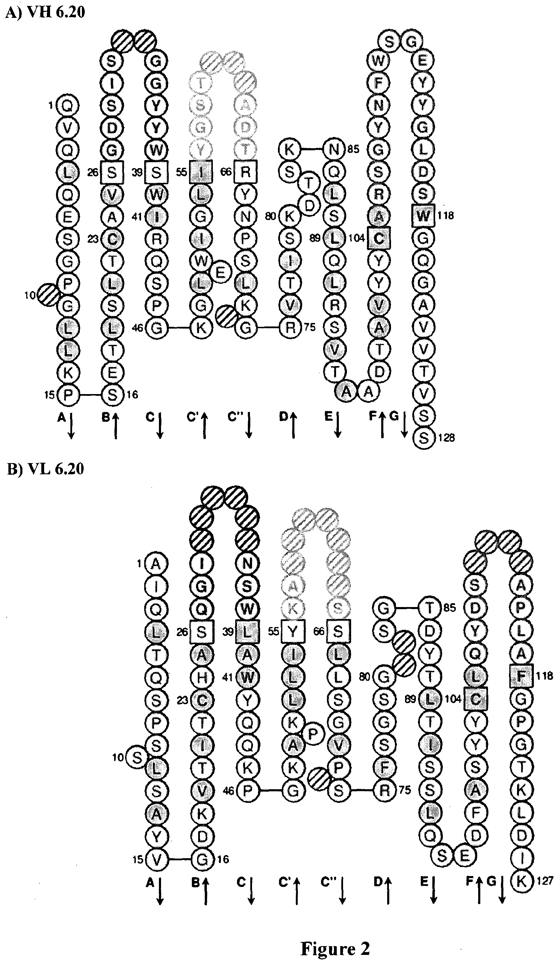

La présence d'un résidu leucine en position 51 de la SEQ ID NO : 1 correspond à la leucine en position 54 sur la figure 2A.
La présence d'un résidu glycine en position 67 de la SEQ ID NO : 1 correspond à la glycine en position 74 sur la figure 2A.
La présence d'un résidu leucine en position 55 de la SEQ ID NO : 2 correspond à la leucine en position 68 sur la figure 2B.

De préférence, l'invention concerne une immunoglobuline de classe G (IgG) dirigée contre l'antigène protecteur (PA) de la toxine du charbon, ou un de ses fragments, comprenant :
- une région variable de chaîne lourde comprenant la séquence SEQ ID NO : 1, et/ou
- une région variable de chaîne légère comprenant la séquence SEQ ID NO : 2.

Les constantes de cinétique de l'interaction entre l'immunoglobuline de l'invention et l'antigène PA, dont la constante d'affinité (K_{D}), peuvent être déterminées par les techniques conventionnelles connues de l'homme du métier, notamment selon la méthode décrite dans l'exemple 4.

Dans un mode de réalisation particulier, l'invention concerne une immunoglobuline ou un de ses fragments telle que définie ci-dessus, dans laquelle :
- la région constante de ladite chaîne lourde comprend, ou consiste en, une séquence d'acides aminés représentée par la séquence SEQ ID NO : 3, et
- la région constante de chaîne légère comprend, ou consiste en, une séquence d'acides aminés représentée par la séquence SEQ ID NO : 4.

L'invention concerne une immunoglobuline telle que définie ci-dessus, dans laquelle :
- chacune des chaînes lourdes comprend, ou consiste en, une séquence d'acides aminés représentée par SEQ ID NO : 5, et
- chacune des chaînes légères comprend, ou consiste en, une séquence d'acides aminés représentée par SEQ ID NO : 6.
Une telle immunoglobuline est appelée 35PA83 «6.20» dans la présente demande.

Dans un mode de réalisation, l'invention concerne une immunoglobuline ou un de ses fragments telle que définie ci-dessus, dans laquelle ladite chaîne lourde et/ou ladite chaîne légère est liée à un peptide signal.

Il est à noter que le peptide signal utilisé dans l'invention peut être différent du peptide signal naturellement associé à une protéine déterminée. L'utilisation d'un peptide signal permet d'augmenter le taux de sécrétion d'un polypeptide d'intérêt.

Un tel peptide signal peut par exemple être optimisé de manière à augmenter le taux de sécrétion de l'immunoglobuline de l'invention.

Ainsi, un peptide signal connu de l'homme du métier peut être utilisé, comme par exemple un peptide signal tel que décrit dans le document WO2011/114063.

Dans un mode de réalisation particulier, le peptide signal consiste en la séquence d'acides aminés suivante : MRWSWIFLLLLSITSANA (SEQ ID NO : 19).

Dans un mode de réalisation avantageux, l'invention concerne une immunoglobuline telle que définie ci-dessus, dans laquelle :
- chacune des chaînes lourdes comprend, ou consiste en, une séquence d'acides aminés représentée par SEQ ID NO : 7, et
- chacune des chaînes légères comprend, ou consiste en, une séquence d'acides aminés représentée par SEQ ID NO : 8.

Les séquences de référence des chaînes, ou fragments de chaînes, lourdes et légères de l'immunoglobuline de l'invention sont présentées dans le tableau 1 ci-dessous.

**Tableau 1. Le peptide signal est surligné. La région variable est indiquée en italique.**

| | |
|---|---|
| SEQ ID NO: 1 Région variable de la chaîne lourde | |
| | |
| SEQ ID NO: 2 Région variable de la chaîne légère | |
| SEQ ID NO: 3 Région constante de la chaîne lourde | |
| SEQ ID NO: 4 Région constante de la chaîne légère | |
| SEQ ID NO: 5 Chaîne lourde complète sans peptide signal | |
| SEQ ID NO: 6 Chaîne légère complète sans peptide signal | |
| SEQ ID NO: 7 chaîne lourde avec peptide signal | |
| SEQ ID NO: 8 chaîne légère avec peptide signal | |

Les régions constantes de chacune des chaînes légères et de chacune des chaînes lourdes de l'anticorps utilisés dans l'invention peuvent être des régions constantes humaines.
Ce mode de réalisation de l'invention permet de diminuer l'immunogénicité de l'anticorps chez l'homme et par là même d'améliorer son efficacité lors de son administration thérapeutique chez l'homme. En outre, les régions constantes humaines permettent d'assurer une demi-vie plus importante chez l'homme, via l'interaction aux récepteurs FcRn.
De manière préférée, les régions constantes de chacune de chaînes légères de l'immunoglobuline de l'invention sont de type K.
Alternativement, la région constante de chacune des chaînes légères de l'immunoglobuline de l'invention est de type λ.

Dans un mode de réalisation particulier, et notamment lorsque les régions constantes de chacune des chaînes légères et de chacune des chaînes lourdes de l'immunoglobuline de l'invention sont des régions humaines, la région constante de chacune des chaînes lourdes de l'anticorps peut être de type γ1, de type γ2, de type γ3, ou encore de type y 4.

Dans un mode de réalisation préféré, l'invention concerne une immunoglobuline ou un de ses fragments telle que définie ci-dessus, dans laquelle la région constante de chacune des chaînes lourdes est de type γ1.

Dans un mode de réalisation, l'invention concerne une immunoglobuline ou un de ses fragments telle que définie ci-dessus, possédant sur son site de glycosylation Asn297 des N-glycannes dont le taux de fucosylation est inférieur à 65%, de préférence inférieur à 50%, de préférence encore inférieur à 40%.

Dans un mode de réalisation, la présente invention concerne une immunoglobuline de classe G (IgG) dirigée contre l'antigène protecteur (PA) de la toxine du charbon, ou un de ses fragments, comprenant :
- une région variable de chaîne lourde comprenant une séquence d'acides aminés possédant au moins 90% d'identité en acides aminés avec la séquence SEQ ID NO : 1, de préférence comprenant la séquence SEQ ID NO : 2, étant entendu qu'elle comprend les acides aminés Leucine en position 51 et Glycine en position 67, et
- une région variable de chaîne légère comprenant une séquence d'acides aminés possédant au moins 90 % d'identité en acides aminés avec la séquence SEQ ID NO : 2, de préférence comprenant la séquence SEQ ID NO : 2 étant entendu qu'elle comprend la Leucine en position 55,
ladite immunoglobuline ou un de ses fragments possédant une constante d'affinité (K_{D}) vis-à-vis dudit antigène protecteur de la toxine du charbon inférieure à 2.10⁻⁹M, de préférence inférieure ou égale à 3,3.10⁻¹¹M,
ladite immunoglobuline possédant sur son site de glycosylation Asn297 des N-glycannes dont le taux de fucosylation est inférieur à 65%, de préférence inférieur à 50%, de préférence encore inférieur à 40%.

Dans un mode de réalisation particulier, l'invention concerne une immunoglobuline ou un de ses fragments telle que définie ci-dessus, dans laquelle :
- la région constante de ladite chaîne lourde comprend, ou consiste en, une séquence d'acides aminés représentée par la séquence SEQ ID NO : 3, et
- la région constante de chaîne légère comprend, ou consiste en, une séquence d'acides aminés représentée par la séquence SEQ ID NO : 4,
ladite immunoglobuline ou un de ses fragments possédant une constante d'affinité (K_{D}) vis-à-vis dudit antigène protecteur de la toxine du charbon inférieure à 2.10⁻⁹M, de préférence inférieure ou égale à 3,3.10⁻¹¹M,
ladite immunoglobuline possédant sur son site de glycosylation Asn297 des N-glycannes dont le taux de fucosylation est inférieur à 65%, de préférence inférieur à 50%, de préférence encore inférieur à 40%.

Dans un mode de réalisation particulier, l'invention concerne une immunoglobuline telle que définie ci-dessus, dans laquelle :
- chacune des chaînes lourdes comprend, ou consiste en, une séquence d'acides aminés représentée par SEQ ID NO : 5, et
- chacune des chaînes légères comprend, ou consiste en, une séquence d'acides aminés représentée par SEQ ID NO : 6,
ladite immunoglobuline possédant une constante d'affinité (K_{D}) vis-à-vis dudit antigène protecteur de la toxine du charbon inférieure à 2.10⁻⁹M, de préférence inférieure ou égale à 3,3.10⁻¹¹M,
ladite immunoglobuline possédant sur son site de glycosylation Asn297 des N-glycannes dont le taux de fucosylation est inférieur à 65%, de préférence inférieur à 50%, de préférence encore inférieur à 40%.

Dans un mode de réalisation particulier, l'invention concerne une immunoglobuline telle que définie ci-dessus, dans laquelle :
- chacune des chaînes lourdes comprend, ou consiste en, une séquence d'acides aminés représentée par SEQ ID NO : 7, et
- chacune des chaînes légères comprend, ou consiste en, une séquence d'acides aminés représentée par SEQ ID NO : 8,
ladite immunoglobuline possédant une constante d'affinité (K_{D}) vis-à-vis dudit antigène protecteur de la toxine du charbon inférieure à 2.10⁻⁹M, de préférence inférieure ou égale à 3,3.10⁻¹¹M,
ladite immunoglobuline possédant sur son site de glycosylation Asn297 des N-glycannes dont le taux de fucosylation est inférieur à 65%, de préférence inférieur à 50%, de préférence encore inférieur à 40%.

Les immunoglobulines de l'invention se distinguent donc notamment des immunoglobulines anti-toxine du charbon déjà connues par leur faible taux de fucosylation.

De plus, les immunoglobulines de l'invention présentent également un profil de N-glycosylation particulier.

Dans un mode de réalisation particulier, l'invention concerne une immunoglobuline ou un de ses fragments telle que définie ci-dessus, possédant sur son site de glycosylation Asn297 une structure glycannique de type biantenné, avec des chaînes courtes, une faible sialylation, présentant des mannoses terminaux et/ou des N-acétylglucosamines terminaux non intercalaires.

Les formes glycosylées envisageables pour les immunoglobulines selon l'invention sont présentées dans le tableau 2 suivant.

**Tableau 2.**

| **Symbole** | **Forme glycosylée** |
|---|---|
| G0 | Complexe biantenné avec structure agalactosylée |
| G1 | Complexe biantenné avec une structure comprenant un seul galactose |
| G0F | Complexe biantenné avec structure agalactosylée (G0), dans lequel le noyau pentasaccharidique est substitué par un fucose |
| G1F | Complexe biantenné avec une structure comprenant un seul galactose (G1), dans lequel le noyau pentasaccharidique est substitué par un fucose |

Dans un mode de réalisation, l'invention concerne une immunoglobuline ou un de ses fragments telle que définie ci-dessus, présentant une teneur supérieure à 60 % pour les formes G0+G1+G0F+G1F, la teneur des formes G0F+G1F étant inférieure à 50 %.

Dans un mode de réalisation, l'invention concerne une immunoglobuline ou un de ses fragments telle que définie ci-dessus, présentant une teneur supérieure à 60 % pour les formes G0+G1+G0F+G1F, la teneur en fucose étant inférieure à 65 %.

Dans un mode de réalisation, l'invention concerne une immunoglobuline ou un de ses fragments telle que définie ci-dessus, présentant une teneur inférieure à 40% pour les formes G0F+G1F.

Un autre aspect de l'invention est un polynucléotide codant l'immunoglobuline de l'invention ou un de ses fragments.

Ainsi, un autre aspect de l'invention concerne un polynucléotide comprenant au moins une séquence nucléotidique codant la chaîne lourde ou la chaîne légère d'une immunoglobuline ou un de ses fragments telle que définie ci-dessus.

Dans un mode de réalisation particulier, l'invention concerne un polynucléotide comprenant au moins une séquence nucléotidique choisie parmi SEQ ID NO : 9, 10, 11, 12, 13, 14, 15, 16, 17 et 18.

**Tableau 3. Les séquences codantes sont indiquées en gras. Les séquences codant le peptide signal sont surlignées. Les séquences de Kozak sont soulignées. Les codons stop sont en italique et soulignés.**

| | |
|---|---|
| SEQ ID NO : 9 Séquence nucléotidique codant la partie variable de la chaîne lourde | |
| SEQ ID NO : 10 Séquence nucléotidique codant la partie variable de la chaîne légère | |
| SEQ ID NO : 11 Séquence nucléotidique codant la partie constante de la chaîne lourde | |
| SEQ ID NO : 12 Séquence nucléotidique codant la partie constante de la chaîne légère | |
| | |
| SEQ ID NO: 13 Séquence nucléotidique codant la chaîne lourde | |
| SEQ ID NO: 14 Séquence nucléotidique codant la chaîne légère | |
| SEQ ID NO: 15 Séquence nucléotidique codant la chaîne lourde comprenant un peptide signal en N-terminal | |
| | |
| SEQ ID NO: 16 Séquence nucléotidique codant la chaîne légère comprenant un peptide signal en N-terminal | |
| SEQ ID NO : 17 Séquence nucléotidique codant la chaîne lourde telle que synthétisée | |
| | |
| SEQ ID NO: 18 Séquence nucléotidique codant la chaîne légère telle que synthétisée | |

De tels polynucléotides peuvent être insérés dans un vecteur recombinant pour le clonage ou pour l'expression des immunoglobulines de l'invention.

Un autre aspect de l'invention est donc un vecteur comprenant un polynucléotide codant l'immunoglobuline de l'invention ou un de ses fragments.

Le terme *"vecteur"* se réfère à une molécule d'acide nucléique dans lequel une séquence d'intérêt peut être insérée par digestion avec une endonucléase de restriction, puis ligation, pour le transport entre différents environnements génétiques ou pour l'expression dans une cellule hôte. Les vecteurs sont par exemples les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus.

Un vecteur de clonage est un vecteur capable de se répliquer dans une cellule hôte et qui est de plus caractérisé par la présence de un ou plusieurs sites de restriction.
Un vecteur d'expression est un vecteur dans lequel la séquence d'ADN d'intérêt peut être insérée par digestion avec une endonucléase de restriction et ligation de telle façon qu'elle puisse être répliquée et/ou transcrite en ARN.
Les vecteurs peuvent contenir en outre un ou plusieurs marqueurs de sélection ou d'identification des cellules ayant été transformées ou transfectées avec le vecteur.

La présente invention inclut tous les vecteurs recombinants contenant des séquences codantes pour la transformation, transfection ou thérapie génique eucaryote ou procaryote. De tels vecteurs pourront être préparés selon les techniques conventionnelles de biologie moléculaire et comprendront en outre un promoteur approprié, optionnellement une séquence signal pour l'export ou la sécrétion, et des séquences régulatrices nécessaires pour la transcription de la séquence nucléotidique.

Le vecteur de l'invention peut comporter les séquences codant la chaîne lourde et/ou la chaîne légère de l'immunoglobuline de l'invention.

Un mode de réalisation de l'invention concerne donc un vecteur comprenant au moins un polynucléotide tel que défini ci-dessus comprenant au moins une séquence nucléotidique codant la chaîne lourde ou la chaîne légère d'une immunoglobuline telle que définie ci-dessus.

Un autre mode de réalisation de l'invention concerne un vecteur comprenant au moins un polynucléotide tel que défini ci-dessus comprenant au moins une séquence nucléotidique codant la chaîne lourde et une séquence codant la chaîne légère d'une immunoglobuline telle que définie ci-dessus.

Un des vecteurs approprié dans le cadre de l'invention est une molécule d'acide nucléique recombinante adaptée pour recevoir et exprimer un premier polynucléotide et un second polynucléotide, de façon à permettre l'expression d'anticorps hétérodimérique tel qu'un anticorps de longueur complète ou des fragments F(ab')2 ou Fab selon l'invention.
Un tel vecteur fournit un système pour cloner indépendamment les deux polynucléotides dans deux cassettes séparées présentes dans le vecteur, de façon à former deux cistrons séparés pour l'expression d'un premier et d'un second polypeptide de l'anticorps hétérodimérique. Un tel vecteur d'expression est appelé vecteur di-cistronique.

Dans un mode de réalisation particulier, le vecteur est une molécule d'acide nucléique dans laquelle un polynucléotide codant la région variable de chacune des chaînes légères de l'immunoglobuline, et un polynucléotide codant la région constante de chacune des chaînes légères de l'immunoglobuline ont été insérées, afin de les introduire et de les maintenir dans une cellule hôte. Un tel vecteur permet l'expression de ces polynucléotides dans la cellule hôte car il possède des séquences indispensables (promoteur, séquence de polyadénylation, gène de sélection) à cette expression. De tels vecteurs sont bien connus de l'homme du métier, et peuvent être un adénovirus, un rétrovirus, un plasmide ou un bactériophage, cette liste n'étant pas limitative.

Il est par ailleurs possible d'introduire dans un tel vecteur des unités de transcription, c'est-à-dire des polynucléotides contenant des éléments régulateurs nécessaires à la transcription d'un acide nucléique d'intérêt en ARN.

Des unités de transcription connues de l'homme du métier peuvent être utilisées, comme par exemple celles décrites dans le document WO2013/061010.

La cellule hôte productrice de l'immunoglobuline est une caractéristique importante car elle confère à l'immunoglobuline certaines de ses propriétés particulières. En effet, le moyen d'expression des immunoglobulines est à l'origine de modifications post-traductionnelles, notamment des modifications de la glycosylation, qui peuvent varier d'une lignée cellulaire à l'autre, et ainsi conférer des propriétés fonctionnelles différentes à des immunoglobulines ayant pourtant des structures primaires identiques.

Un autre aspect de l'invention concerne donc une cellule hôte comprenant un vecteur tel que défini ci-dessus.

Une telle cellule hôte peut être une cellule procaryote ou eucaryote. Les immunoglobulines de la présente invention peuvent notamment être produites dans des cellules eucaryotes, telles que des cellules YB2/0, CHO ou des hybridomes humains ou murins, ainsi que dans des cellules végétales et des animaux transgéniques.

Dans un mode de réalisation particulier, l'invention concerne une cellule hôte telle que définie ci-dessus, caractérisée en ce qu'il s'agit d'une cellule choisie parmi : SP2/0, YB2/0, IR983F, le myélome humain Namalwa, PERC6, les lignées CHO, notamment CHO-K-1, CHO-Led O, CHO-Led, CHO-Lec13, CHO Pro-5, CHO dhfr-, Wil-2, Jurkat, Vero, Molt-4, COS-7, 293-HEK, BHK, K6H6, NSO, SP2/0-Ag 14 et P3X63Ag8.653.

Dans un autre mode de réalisation préféré, l'immunoglobuline est produite dans l'hybridome de rat YB2/0 (cellule YB2/3HL. P2. G11.16Ag.20, déposée à l'American Type Culture Collection sous le numéro ATCC n° CRL-1662).

De préférence, la lignée cellulaire stable exprimant une immunoglobuline selon l'invention, et plus particulièrement choisie dans le groupe précédemment décrit, a intégré le (ou les) vecteur(s) d'expression de la chaîne lourde et de la chaîne légère tel(s) que précédemment décrit(s).

D'autres méthodes sont connues de l'homme du métier pour produire des immunoglobulines faiblement fucosylées, et peuvent être utilisés pour la préparation des immunoglobulines de l'invention.

De manière non exhaustive, il peut s'agir par exemple de méthodes de préparation d'anticorps dans des cellules cultivées en présence de kifunensine, comme cela est décrit par exemple dans le document US 7,700,321.

Des analogues de fucose peuvent également être introduits dans le milieu de culture de cellules productrices d'anticorps comme décrit dans le document US 2009/0317869.

Un autre moyen de produire des anticorps, peut être l'utilisation, par exemple de cellules pour lesquelles la voie de production des GDP-fucose est inhibée, par exemple par inhibition de l'une au moins des enzymes du cycle de production fucose, telle que décrit par exemple dans le document US 2010/291628 ou US 2009/0228994, le document EP 1,500,698, le document EP 1,792,987 ou encore le document US 7,846,725, cette liste n'étant pas limitative.

Il est également possible d'utiliser des ARN interférent (ARNi) inhibant la 1,6-fucosyltransférase comme décrit dans le document US 7,393,683 ou le document WO 2006/133148.

Il peut également s'agir de méthodes de préparation d'anticorps dans des animaux transgéniques, comme cela est décrit dans le document WO 2007/48077. Il peut également s'agir de méthodes de préparation d'anticorps dans des levures, comme cela est décrit par exemple dans le document WO 02/00879.

Dans le cas où la région Fc de l'anticorps possède 100% d'oligosaccharides non fucosylés, c'est-à-dire quand la région Fc de l'anticorps est totalement dépourvue de fucose, il est possible d'utiliser des méthodes de préparation connues de l'homme du métier, comme par exemple celles décrites dans les documents EP 1,176,195, US 7,214,775, US 6,994,292, US 7,425,449, US 2010/223686, WO 2007/099,988, EP 1,705,251, cette liste n'étant pas limitative. Il peut s'agir par exemple d'une méthode utilisant une cellule hôte exprimant au moins un acide nucléique codant pour l'anticorps de l'invention, et dont la glycosylation est modifiée par délétion du gène codant pour l'a 1,6-fucosyltransférase ou par addition d'une mutation de ce gène pour éliminer l'activité α 1,6-fucosyltransférase, et exprimant à ce titre un anticorps dépourvu de fucose. Il peut également s'agir d'une méthode comprenant la mutation des acides aminés de la partie Fc.

Dans un autre aspect, l'invention concerne une composition, notamment pharmaceutique, comprenant au moins une immunoglobuline, de préférence humaine, ou un de ses fragments telle que définie ci-dessus.

Une telle composition pharmaceutique comprend de façon préférée un véhicule pharmaceutiquement acceptable.
Un tel véhicule correspond au sens de l'invention à un matériel non toxique qui n'interfère pas avec l'efficacité de l'activité biologique des ingrédients actifs de la composition.

Le terme *"pharmaceutiquement acceptable"* se réfère à un matériel non toxique qui est compatible avec un système biologique tel qu'une cellule, une culture cellulaire, un tissu ou un organisme. Les caractéristiques du véhicule dépendront du mode d'administration.

Dans un autre aspect, l'invention concerne une immunoglobuline, de préférence humaine, ou un de ses fragments telle que définie ci-dessus, pour son utilisation comme médicament.

Dans un autre aspect, l'invention concerne une immunoglobuline, de préférence humaine, ou un de ses fragments telle que définie ci-dessus, pour son utilisation dans le traitement ou la prévention de pathologies liées aux toxines du charbon.

Le terme *"prévention"* correspond à la prévention de l'apparition de la maladie chez un sujet, en particulier un humain, chez qui la maladie ne s'est pas encore déclarée.

Le terme *"traitement"* correspond à l'inhibition de cette maladie, *i.e.* l'arrêt de son développement, sa régression, ou à la disparition des symptômes et conséquences de la maladie, ou encore à la disparition des causes de la maladie.

Dans un autre aspect, l'invention concerne une méthode pour la détection *in vitro* d'une toxine du charbon comprenant l'antigène protecteur (PA) dans un échantillon biologique, comprenant :
- la mise en contact de l'échantillon avec au moins une immunoglobuline ou un de ses fragments telle que définie ci-dessus, et
- la détection de la liaison de ladite immunoglobuline ou un de ses fragments comme indicateur de la présence de ladite toxine du charbon.
L'échantillon biologique peut être liquide, par exemple de la salive, de l'urine, du fluide cérébrospinal, du sérum ou du sang, ou solide ou semi-solide, par exemple des tissus ou des matières fécales ou un tissu solide tel qu'utilisé couramment en diagnostic histologique.

L'immunoglobuline de l'invention peut être utilisée *in vitro,* par exemple dans des tests immunologiques dans lesquels elles sont utilisées en phase liquide ou liées à un véhicule de phase solide. Des exemples de véhicules bien connus sont le verre, le polystyrène, le polypropylène, le polyéthylène, le dextran, le nylon, l'amylase, la cellulose naturelle ou modifiée, le polyacrylamide, l'agarose ou la magnétite. Des exemples de tests immunologiques utilisant l'immunoglobuline anti-PA de l'invention sont des radioimmunoessais, des marquages histoimmunologiques, des ELISA, des westernblots, des essais d'immunoprécipitation, des essais d'immuno-diffusion, des essais de fixation du complément, des analyses par cytométrie de flux (FACS) ou encore des analyses par puces à protéines.

L'immunoglobuline de l'invention peut être marquée. Des exemples de marqueurs comprennent les enzymes, les radio-isotopes, les composés fluorescents, les métaux colloïdes, les composés chimioluminescents, et les composés bioluminescents.
Les méthodes de liaison d'un marqueur à une immunoglobuline sont bien connues de l'homme du métier.

Une autre technique de marquage consiste à coupler l'immunoglobuline à des haptènes de faibles poids moléculaire, ces haptènes pouvant être spécifiquement modifiés au moyen d'une seconde réaction. Des exemples d'haptènes sont la biotine, qui réagit avec l'avidine, ou le dinitrophenol, le pyridoxal ou la fluorescéine, qui peuvent réagir avec des immunoglobulines spécifiques anti-haptènes.

Dans un autre aspect, l'invention concerne un kit pour la détection d'une toxine du charbon comprenant l'antigène protecteur (PA), ledit kit comprenant :
- un récipient comprenant au moins une immunoglobuline anti-PA ou un de ses fragments de l'invention et qui peut être ou non marquée,
- optionnellement, un récipient comprenant des solutions tampons,
- et optionnellement un récipient comprenant des moyens de détection de l'immunoglobuline ou un de ses fragments marquée, tel qu'une protéine de liaison à la biotine, par exemple l'avidine ou la streptavidine, liée à une molécule rapporteur, telle qu'un marqueur fluorescent ou enzymatique.
Ce container peut aussi comprendre des moyens de détection de l'immunoglobuline ou un de ses fragments non marquée, soit essentiellement des anticorps ou fragments d'anticorps.

Dans un mode de réalisation, l'invention concerne un kit pour la détection d'une toxine du charbon comprenant l'antigène protecteur (PA) tel que défini ci-dessus, ledit kit comprenant :
- un récipient comprenant au moins une immunoglobuline ou un de ses fragments telle que définie ci-dessus, marquée, et
- un récipient comprenant des moyens de détection de ladite immunoglobuline ou un de ses fragments marquée.

La quantité d'immunoglobuline marquée administrée doit être suffisante pour permettre la détection de la liaison de l'immunoglobuline à la toxine. La quantité d'immunoglobuline marquée administrée dépendra de facteurs tels que l'âge et le sexe du sujet, ainsi que du stade de la maladie. La quantité administrée peut varier entre 0,01 mg/kg et 50 mg/kg, préférablement entre 0,1 mg/kg et 20 mg/kg, et plus préférablement entre 0,1 mg/kg et 2 mg/kg.

L'immunoglobuline de l'invention peut être liée à un radioisotope directement, ou indirectement, par l'intermédiaire de groupes fonctionnels. Des groupes fonctionnels couramment utilisés sont par exemple l'acide diéthylène-triaminepentacétique (DTPA) et l'acide éthylène-diamine-tétraacétique (EDTA). Des exemples d'ions métalliques radioisotopes sont ¹¹¹In, ⁹⁷Ru, ⁶⁷Ga, ⁶⁸Ga, ⁷²As, ⁸⁹Zr et ²⁰¹Tl.
Les immunoglobulines de l'invention peuvent aussi être marquées avec un isotope paramagnétique pour le diagnostic par imagerie de résonance magnétique (IRM) ou par résonance de spin électronique (ESR). Des radioisotopes gamma émetteurs de positrons peuvent également être utilisés, tels que ¹⁵⁷Gd, ⁵⁵Mn, ¹⁶²Dy, ⁶⁸Ga, ⁵²Cr et ⁵⁶Fe.

Les immunoglobulines ou un de ses fragments de l'invention peuvent également être utilisées *in vitro* ou *in vivo* pour suivre l'évolution du traitement de la maladie, par exemple en déterminant l'augmentation ou la diminution du nombre de cellules ciblées par les toxines du charbon ou les changements dans la concentration de la toxine PA dans un échantillon biologique.

Dans un autre aspect, la présente invention concerne également une méthode de traitement d'un sujet, de préférence un humain, susceptible d'être infecté par *Bacillus anthracis,* dans laquelle une quantité thérapeutiquement efficace d'une immunoglobuline selon l'invention est administrée audit sujet.

Le terme *"quantité thérapeutiquement efficace"* se réfère à la quantité qui est suffisante pour effectuer le traitement lorsqu'elle est administrée à un sujet qui nécessite un tel traitement. La quantité thérapeutique effective dépend du sujet, du stade de la maladie à traiter et du mode d'administration, et peut être déterminée par des opérations de routine par l'homme du métier.

Le terme *"charbon"* se réfère à toute maladie causée, directement ou indirectement, par une infection par *Bacillus anthracis.*

Les symptômes initiaux d'une infection par inhalation ressemblent à ceux d'un rhume ou d'une grippe (fièvre, douleurs musculaires). Après plusieurs jours, ces symptômes progressent vers des problèmes sévères de détresse respiratoire et de choc septique. Sans aucun traitement, l'inhalation de spores de *Bacillus anthracis* est en générale fatale. L'infection cutanée par le charbon a lieu lorsque la bactérie entre dans la peau au niveau d'une brèche cutanée préexistante. Cette infection donne lieu dans un premier temps à une papule, qui se développe en deux à trois jours en une vésicule puis en un ulcère de 1 à 3 centimètres de diamètre présentant une aire nécrotique au centre. L'infection gastro-intestinale par le charbon se développe suite à la consommation de viande contaminée et est caractérisée par une inflammation aigue du tractus intestinal.

Une quantité thérapeutiquement efficace correspond à une quantité suffisante pour diminuer les symptômes de la maladie et l'évolution de l'infection. Cette quantité peut varier avec l'âge, le sexe du sujet et le stade de la maladie et sera déterminée par l'homme du métier. Une quantité thérapeutiquement efficace peut varier entre 0,01 mg/kg et 50 mg/kg, préférablement entre 0,1 mg/kg et 20 mg/kg, et plus préférablement entre 0,1 mg/kg et 2 mg/kg, en une ou plusieurs administrations quotidiennes, pendant un ou plusieurs jours, de préférence en une ou deux administrations au total.
Le mode d'administration peut être par injection ou par perfusion graduelle. L'injection peut être intraveineuse, intra-péritonéale, intramusculaire, sous-cutanée ou transdermale.
Les préparations pour une administration parentérale peuvent inclure des solutions aqueuses ou non-aqueuses stériles, des suspensions ou des émulsions. Des exemples de solvents non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

Dans un autre aspect, l'invention concerne un immunoconjugué comprenant une immunoglobuline ou un de ses fragments telle que définie ci-dessus lié à un agent thérapeutique.

Dans un mode de réalisation, l'invention concerne un immunoconjugué comprenant une immunoglobuline ou un de ses fragments telle que définie ci-dessus liée, de façon directe ou indirecte, à un agent thérapeutique.

De tels agents thérapeutiques comprennent des agents chimiques, des radionucléides, des agents immunothérapeutiques, des cytokines, des chimiokines, des toxines ou des inhibiteurs d'enzyme. Des exemples de toxines sont la chaîne A de la diphtérie, la chaîne A de l'exotoxine, la chaîne A de la ricine, la chaîne A de l'abrine, la chaîne A de la modeccin, l'alphasarcin, les protéines Aleurites fordii, les protéines dianthines, les protéines Phytolaca americana, l'inhibiteur momordica charantia, la curcine, la crotine, l'inhibiteur sapaonaria officinalis, la gélonine, la mitogélline, la restrictocine, la phénomycine, l'énomycine et le tricothecenes. Des exemples de radionucléide sont ²¹²Bi, ¹³¹I, ¹³¹In, ⁹⁰Y et ¹⁸⁶Re.

Avantageusement, l'immunoglobuline de l'invention ou un de ses fragments est associée à un traitement prophylactique avec la tétracycline. L'immunoglobuline de l'invention permet de raccourcir la prophylaxie basée sur la tétracycline, en arrêtant, de manière sécurisée, après un risque d'exposition, par un traitement rapide ("court traitement") de tétracycline suivi d'une injection de l'immunoglobuline de l'invention.

Avantageusement, l'immunoglobuline de l'invention est associée à un traitement curatif avec la ciprofloxacine.

La présente invention sera mieux comprise à l'aide des figures et des exemples suivants.

### LEGENDES DES FIGURES

**Figure 1****.**
   Schéma en collier de perles de la région variable de la chaîne lourde (A) et de la chaîne légère (B) de l'immunoglobuline 35PA83.
   La représentation IMGT en collier de perles est réalisée en accord avec la numérotation IMGT. Les cercles hachurés correspondent aux positions manquantes de la numérotation IMGT.
**Figure 2****.**
   Schéma en collier de perles de la région variable de la chaîne lourde (A) et de la chaîne légère (B) de l'immunoglobuline 35PA83 «6.20».
   La représentation IMGT en collier de perles est réalisée en accord avec la numérotation IMGT. Les cercles hachurés correspondent aux positions manquantes de la numérotation IMGT.
**Figure 3****.**
   Profil HILIC-UPLC/FD des N-glycannes de l'anticorps 35PA83 « 6.20» libérés après un traitement à l'aide de la peptidyl-N-glycosidase F (PNGase F).
   L'axe des abscisses correspond au temps d'élution en minutes. L'axe des ordonnées indique l'intensité relevée pour chaque composé identifié en unités d'émission.

### EXEMPLES

### Exemple 1 : Construction du vecteur codant l'immunoglobuline 35PA83 «6.20»

### - Souches d'Escherichia coli

Les souches d'E. *coli* suivantes ont été utilisées :
- XL1 (Stratagène, La jolla, CA) : recA1 endA1 gyrA96 thi-1 hsdR17 sup E44 relA1 lac [F'proAB laclqZAM15 Tn10(Tetr)].
- SURE (Stratagène): e14(McrA) Δ(mcrCB-hsdSMR-mrr)171 endAl supE44 thi-1 gyrA96 relA1 lac recB recJ sbcC umuC::Tn5 (Kanr) uvrC [F'proAB laclqZΔM15 Tn10 (Tetr)]
- HB2151 (Carte *et al.,* 1985), utilisée pour l'expression de Fabs solubles
- TOP10 (Invitrogen) : utilisée pour la construction du vecteur d'expression eucaryote.

### Toxines

Les toxines du charbon (PA83, LF et EF) ont été achetées chez List laboratories.

### Construction de Fab mutants partir de 35PA83

Un variant a d'abord été construit afin d'humaniser le fragment d'immunoglobuline 35PA83. Ce variant a été obtenu en effectuant les mutations décrites dans les tableaux ci-dessous :

**Tableau 4 : Mutations pour l'humanisation de la chaîne lourde de 35PA83**

| Position de l'acide aminé | 35PA83 | Hu35PA83 |
|---|---|---|
| 1 | Aucun | Q |
| 2 | Aucun | V |
| 3 | Aucun | Q |
| 4 | Aucun | L |
| 5 | Aucun | Q |
| 6 | Aucun | E |
| 7 | Aucun | S |

**Tableau 5 : Mutations pour l'humanisation de la chaîne légère de 35PA83**

| Position de l'acide aminé | 35PA83 | Hu35PA83 |
|---|---|---|
| 1 | Aucun | A |
| 2 | Aucun | I |
| 3 | Aucun | Q |
| 4 | Aucun | L |

A partir de ce variant humanisé, des séquences mutantes dérivées du gène 35PA83 ont ensuite été générées par Massive mutagenesis® (Biomethodes, Evry, France). Les mutations ont été introduites dans les CDRs des chaînes lourde et légère en utilisant des codons NNS (N code pour A, T, G, ou C et S code pour G ou C). Les régions CDRs ont été définies selon Kabat et al. (Wu and Kabat, 1970) et IMGT (Lefranc, Pommie *et al.,* 2003).
L'ADN obtenu a été utilisé pour transformer les cellules SURE par électroporation. Après ajout de milieu SB supplémenté en carbénicilline à la culture et incubation pendant 1h à 37°C, 1 ml de phage helper VCSM13 (Andris-Widhopf et al., 2001) (environ 1012 pfu) a été ajouté à la culture. Après incubation pendant 2h, 70 µg/ml de kanamycine ont été ajoutés et la culture a été mise sous agitation pendant la nuit.

### Sélection d'immunoglobulines par phage

Les particules de phage-immunoglobuline ont été purifiées et concentrées à partir de 50 ml de culture par précipitation au polyéthylène glycol (PEG), puis re-suspendues dans 3 ml de PBS-BSA 1 %- azide 0,02% et filtrées sur un filtre de 0,45 µm. Le titre de cette préparation de phage était d'environ 1010 pfu/ml. Les phages-immunoglobulines ont été soumis à trois cycles d'infection-sélection-récupération, correspondant à 5, 10 et 15 lavages respectivement, tel que précédemment décrits (Andris-Widhopf, Rader *et al.,* 2000).

### Expression, extraction périplasmique et purification de Fab mutants solubles

Chaque variant ADN a été transformé dans des bactéries de la souche d'E. *coli* HB2151, rendues chimiquement compétentes. Les cellules ont été cultivées à 30°C, agitées à 250 rpm dans 1 L de milieu SB contenant 50 µg/ml de carbénicilline et 0,1 % de glucose. Lorsque la culture a atteint une absorbance à λ= 600 nm de 1,5, une induction avec 1 mM d'IPTG (Isopropyl β-D-1-thiogalactopyranoside) a été effectuée pendant 18h à 22°C.
Les fragments d'immunoglobulines ont été extraites avec du sulfate de polymixine B (Sigma) et purifiés sur colonne de nickel (Ni-NTA spin column, QIAGEN, Valencia, CA) selon les instructions du fabricant, puis dialyses avec du PBS 1 X à 4°C pendant 3h.

### Quantification du Fab soluble

La pureté du Fab a été testée par SDS-PAGE et sa concentration a été déterminée à l'aide du logiciel Quantity One(R) (Biorad).

### Mesure en temps réel de la résonance plasmonique de surface (SPR)

Les constantes de cinétique de l'interaction entre PA83 et les variants 35PA83 ont été déterminées en utilisant le système Biacore X SPR (BIAcore, Uppsala, Sweden). Le PA83 a été immobilisé sur une puce sensible CM5 (Biacore) en utilisant une procédure de couplage des aminés par injection de 30 µl de 2 µg/ml de PA83 dans 10 mM de sodium acétate pH 4,5. Pour minimiser la probabilité de reliaison, le K_{D} a été mesuré en utilisant un débit élevé (30 µl/min) et une quantité minimale d'antigène couplé (environ 500 RU, unités de résonance). Le taux de liaison de différentes concentrations de Fab allant de 5 à 400 nM dans du PBS a été déterminé à un débit de 30 µl/min. Les données de liaison ont été introduites dans un modèle langmuir 1:1 du logiciel d'évaluation BIA (Biacore). Les constantes d'association et de dissociation (kₒₙ et k_{off} respectivement) pour la liaison du Fab à PA83 ont été déterminées à 35°C.

### Analyse de séquences

Les séquences des chaînes lourde et légère des clones sélectionnés ont été déterminées par Génome Express (Meylan, France) en utilisant les amorces ompseq et newpelseq (Andris-Widhopf, Rader et al., 2000). Les séquences ont été analysées en ligne, en utilisant le système IMGT.

Parmi les clones sélectionnés, le variant 35PA83 « 6.20 » a été identifié.
La région variable de la chaîne lourde (VH) du variant «6.20» (SEQ ID NO : 1) présente 2 mutations comparée à celle du Fab 35PA83 humanisé : H→L (résidu 54 selon la nomenclature d'IMGT) et S→G (résidu 74 selon la nomenclature d'IMGT).
La région variable de la chaîne légère (VL) du variant «6.20» (SEQ ID NO : 2) présente 1 mutation comparée à celle du Fab 35PA83 humanisé : Q→L (résidu 68 selon la nomenclature d'IMGT).

### Construction du vecteur d'expression ATH-GA pour l'expression de l'immunoglobuline 35PA83 « 6.20 »

Une séquence codant le peptide signal optimisé MB7 (SEQ ID NO:19, **MRWSWIFLLLLSITSANA**) a été ajoutée en N-terminal des deux séquences codant respectivement les parties variables de la chaîne lourde et la chaîne légère du variant 35PA83 « 6.20 » (SEQ ID NO : 7 et 8). Ces séquences ont été optimisées et synthétisées par GeneArt (Regensburg, Germany).

Les séquences des chaînes lourde et légère du variant ont ensuite été clonées dans le vecteur *HK gen EFss* pour obtenir le vecteur ATH-GA
Le séquençage du vecteur ATH-GA a été réalisé selon la technique de Sanger (ou méthode des terminateurs de chaîne, réf. : Sanger F. et al, 1977, PNAS 74 : 5463).
Les séquençages ont été réalisés par Eurofins MWG Operon (Ebersberg, Germany) selon le niveau de qualité réglementaire« GLP ». Il s'agit du niveau de qualité maximal, avec couverture double brin de la séquence d'ADN, un minimum de redondance de 2 fois, une précision supérieure à 99,999%, des instruments dédiés, l'édition d'un rapport de qualité et l'archivage des documents générés.
La préparation du vecteur ATH-GA a été conservée en tampon TE (10 mM Tris pH 8 et 1 mM EDTA) à -20°C avant ajustement à la concentration de 1 µg/µl pour transfection dans les lignées cellulaires YB2/0.

### Exemple 2 : Obtention de transformants producteurs de l'immunoglobuline 35PA83 « 6.20 »

L'anticorps 35PA83 «6.20» a été produit dans les lignées YB2/0. Pour les expérimentations ci-dessous, la technique ELISA mise en oeuvre se fait selon les conditions suivantes :
Des plaques de 96 puits microtitrés (maxisorp, Nunc, Danemark) sont coatées avec du PA dilué dans du PBS (5 µg/ml, 100 µl par puits), durant la nuit à 4°C. Les plaques sont bloquées par ajout de 200 µl de PBS-BSA 5% à 37°C pendant 1 heure, et des sérums dilués en série dans du PBS-Tween 20 0,1% - BSA 1% sont incubés (100 µl par puits) à 37°C pendant 2 heures. Une phosphatase alcaline anti-IgG de souris conjuguée ou une "anti-human IgG alkaline phosphatase conjugate " (Sigma) sont incubées (1/10 000) à 37°C pendant 1 heure. Un substrat P-Nitrophenyl Phosphate est ensuite incubé pendant 30 minutes à température ambiante. Les résultats sont déterminés par mesure de l'absorbance à 405 nm avec un lecteur automatisé de microplaques (iEMS reader MF, Labsystems, Helsinki, Finlande). Le dernier point de dilution dont la réversion détermine le titre du sérum, est déterminé comme donnant un signal inférieur ou égal à 2 fois le sérum naïf utilisé pour le contrôle négatif.

### a. Taux de transformation

► Le vecteur ATH-GA a été introduit par électroporation dans la lignée cellulaire hôte YB2/0. Après mise en milieu sélectif (avec l'agent sélectif G418), des pools de transfectants ont été obtenus et étalés en milieu semi-solide en présence d'anticorps anti-IgG humaines fluorescent et dans des conditions permettant la croissance de colonies isolées. L'intensité de fluorescence des colonies, proportionnelle à leur capacité de production, a été analysée par l'automate ClonePixFL (CPFL) et les colonies présentant la fluorescence la plus importante ont été reprises par l'automate.

### b. Sélection des transformants

### ► Taux de production : premier dépistage des transformants plus forts producteurs

La production d'IgG humaines a été déterminée par la technique ELISA sur les surnageants des puits de P96 en double sélection contenant de cellules afin de réaliser une première hiérarchisation des cloïdes sur leurs capacités de production.
Trois dépistages successifs (tous les 2-3 jours) ont été réalisés et les 10 meilleurs producteurs de chaque dépistage ont été sélectionnés. Sur 528 transformants, 27 ont été poursuivis et entretenus en P24 et une étude de leur productivité à J+3 et de leur production maximale (J+7) a été effectuée en parallèle.

### ► Productivité à J+3 et production maximale (J+7)

Les meilleurs clones producteurs sélectionnés avec une productivité en majorité supérieure à 5 pcd et une production maximale supérieure à 10 µg/ml ont été amplifiés cellulairement en milieu sélectif (double sélection) pour sauvegarde en azote liquide.

### c. Sélection d'un clone et production de l'immunoglobuline 35PA83 «6.20» en cytoculteur

Un clone a été retenu pour la production de l'immunoglobuline 35PA83 «6.20» en cytoculteur (10L) en fonction de ses caractéristiques de croissance et productivité. L'immunoglobuline 35PA83 a été produite, concentrée et purifiée.

### Exemple 3 : Profil de N-glycosylation de 35PA83 « 6.20 »

Un dosage du taux de fucose sur les surnageants des cloïdes sélectionnés à J+3 et J+7 a été effectué par la technique ELISA.
Pour déterminer le profil de N-glycosylation des immunoglobulines 35PA83 «6.20», celles-ci ont été traitées par voie enzymatique à l'aide de la peptidyl-N-glycosidase (PNGase F). Le profilage quantitatif du mélange de N-glycannes a ensuite été réalisé par chromatographie liquide ultra-performance de partage en mode HILIC (UPLC/FD).

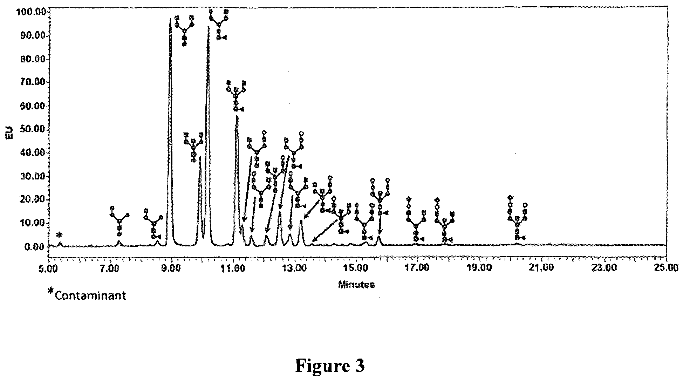

Résultats : La figure 3 montre le profil de N-glycosylation des immunoglobulines 35PA83 «6.20». Le tableau 6 recense les espèces majeures identifiées, affectées de leur rapport molaire relatif (RMR, %), les taux de fucosylation (Fuc), de GlcNAc intercalaire (Bis-GlcNAc) et de sialylation, ainsi que l'indice de galactosylation (Gal).

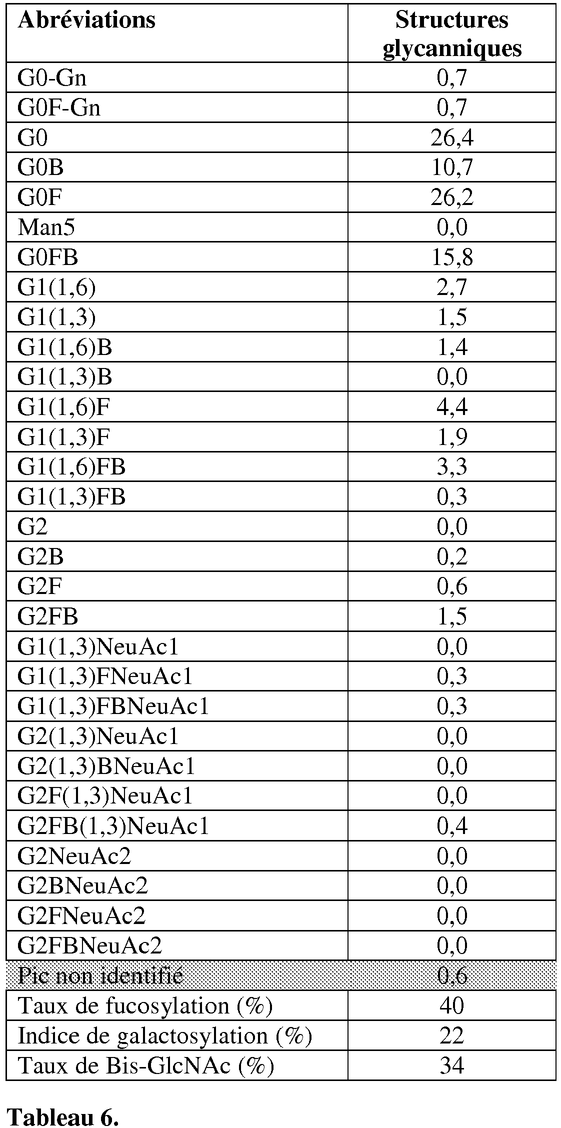

L'immunoglobuline 35PA83 se caractérise par un profil de glycosylation strictement de type complexe biantennée avec des structures majoritairement agalactosylées de type GO (≈80%) dont le noyau pentasaccharidique est substitué ou non par un résidu de fucose (G0F), un résidu de GlcNAc intercalaire (G0B), voire les deux (G0FB). Des structures mono- et bigalactosylées de type G1,2(F)(B) sialylées ou non sont également observées en faible abondance (≈80%), avec un indice de galactosylation des N-glycannes de 22%. Le taux de fucosylation des N-glycannes est de 40%, tandis que le taux de structures à GlcNAc intercalaire est de 34%.

### Exemple 4 : Mesure des constantes cinétiques de l'immunoglobuline 35PA83 « 6.20»

Après la production d'immunoglobuline 35PA83 «6.20» dans des cellules YB2/0, le surnageant de culture cellulaire est récupéré, concentré 15 fois puis soumis à une chromatographie d'affinité au moyen d'une protéine recombinante de A-Sépharose. Une seconde étape de purification est mise en oeuvre au moyen de la colonne échangeuse de cation HiPrep 16/10 SP FF. L'intégrité de l'immunoglobuline purifiée et l'absence de contaminant sont contrôlés par SDS-PAGE et par ELISA pour la liaison au PA83 recombinant.
Les constantes d'affinité sont mesurées par résonance de surface plasmonique (SPR) au moyen du BIAcore(TM) (Biacore Uppsala, Suède). PA83 (List biological laboratories, Campbell, CA) est immobilisé à un maximum de 210 RU sur une puce CM5 (Biacore) au moyen d'une liaison amine, selon les instructions du fournisseur. Un flux de 30 µl/min est maintenu durant la mesure. Pour chaque mesure, un minimum de 6 dilutions d'immunoglobuline dans du tampon HBS-EP (Biacore), avec des concentrations comprises entre 10 à 0,1 µg/ml, sont testées durant 1900 secondes. Après chaque dilution d'immunoglobuline, la puce est régénérée avec de la glycine pH 1,5 (Biacore), avec un flux de 10 µl/min pendant 30 secondes. Les constantes d'affinité sont calculées au moyen d'une méthode d'analyte bivalent (Karlsson *et al.* 1991), et vérifié avec des tests de consistence interne (Schuck *et al.* 1996).

Résultats : L'immunoglobuline 35PA83 «6.20» présente vis-à-vis de l'antigène PA83 une constante d'association (Ka) de 2,93.10⁵ M, une constante de dissociation (K_{d}) de 9,70.10⁻⁶ M et une constante d'affinité (K_{D}) de 3,3.10⁻¹¹ M.

### Exemple 5 : Mesure de l'effet neutralisant de 35PA83 « 6.20 »

Le test de neutralisation *in vitro* est réalisé selon le protocole décrit par Little *et al.* (Little et al., 1990). La lignée cellulaire de macrophage de souris J774A.1 (ATCC-TIB67) est cultivée en flacon de culture non traités pour se développer en suspension. Les cellules sont repiquées 2 fois par semaine en milieu IMDM 10% FCS à 0.3x10⁶ cellules/mL.
L'activité toxique est obtenue par mélange extemporané de PA (List laboratories) et de LF (List laboratories). Une suspension cellulaire à 5x10⁵ cellules/mL en IMDM sans rouge de phénol contenant 5% de FCS est distribuée en microplaque à fond rond à raison de 200%µL/puits. Après 16h à 37°C 5% CO2, les cellules adhérentes forment un tapis de cellules en monocouche.

### J0 :

- Elimination du milieu de culture (élimination de la LDH libérée spontanément lors de la culture)
- Préparation des gammes de concentration de PA 100ng/mL constant + LF concentration variable (100-50-25-12.5-6.25-3.12-1.6-0.78 ng/mL)
- Préparation des gammes de concentration de LF 100ng/mL constant + PA concentration variable (100-50-25-12.5-6.25-3.12-1.6-0.78 ng/mL)
- 200µL de chacune des concentrations de mélange de toxine sont distribués par puits, la plaque est incubée 4h à 37°C
- Pour tester le pouvoir neutralisant de l'anticorps anti-PA une concentration fixe de mélange toxique est choisie et est préalablement incubée pendant 1h à 37°C avec une série de dilution de l'anticorps, puis distribuée dans la plaque
- Une gamme de lyse cellulaire induite par 1% Triton X100 et représentant 100-75-50-25-12.5-6.25 et 0% de lyse
Les échantillons sont déposés en triplicate.

### J0+4h :

La concentration de LDH libérée par la lyse des cellules est mesurée suivant les instructions du kit Cyto Tox 96 Non-radioactive Cytotoxic Assay de chez Proméga.

Les concentrations optimales de PA et LF sont déterminées en réalisant un effet dose de PA en présence d'une concentration fixe de LF et un effet dose de LF en présence d'une concentration fixe de PA.
Les valeurs d'EC50 sont pour PA de 31 et 32ng/mL en présence de 100ng/mL de LF, et pour LF de 6.5 et 6.2ng/mL en présence de 100ng/mL de PA.
La combinaison de 100ng/mL PA avec 100ng/mL LF permet d'obtenir une lyse cellulaire maximale proche de 80%.
L'activité neutralisante de l'anticorps anti-PA est déterminée par sa capacité à se fixer au PA et à ainsi bloquer la liaison de PA aux récepteurs cellulaires.
La toxine, constituée du mélange 100ng/mL de PA et 100ng/mL de LF est incubée en présence de différentes concentrations d'anticorps anti-PA pendant 1h à 37°C. 200µL du mélange sont ensuite déposés en triplicate dans les puits contenant les cellules J774A.1.

Résultats : L'effet neutralisant de l'anticorps 35PA83 « 6.20 » a été évalué dans 2 séries d'essais en conditions suivantes :
Série 1 : concentration d'anticorps anti-PA : 50-10-2-0.4-0.08-0.016-0.0032-0ng/mL
Série 2 : concentration d'anticorps anti-PA : 50-15.01-4.51-1.35-0.406-0.1220-0.037-0ng/mL
Dans ces conditions, l'anti-PA neutralise de façon dose-dépendante l'effet de la toxine PA/LF et inhibe complètement la mortalité cellulaire à des concentrations supérieures à 10ng/mL.
La valeur de 50% de neutralisation (EC₅₀) est déterminée pour l'immunoglobuline 35PA83 «6.20» ; elle est autour de 4-5ng/mL.

### Exemple 6 : Etudes pharmacocinétiques

Pour évaluer le temps de demi-vie de l'immunoglobuline 35PA83 «6.20», six souris A/J âgées de six semaines (Harlan, Gannat, France) ont été réparties en deux sous-groupes de taille égale. Toutes les souris ont reçu l'immunoglobuline 35PA83 «6.20», administrée par une injection unique sous-cutanée à la dose de 10 mg/kg. Le sang a été collecté par ponction rétro-orbitale quotidienne, à partir du jour 1 et jusqu'au jour 6 après l'injection, puis du jour 8 jusqu'au jour 10 après l'injection, en utilisant chaque jour distinct les souris en alternance. Le temps de demi-vie de l'immunoglobuline 35PA83 « 6.20» a été établi à partir des résultats des tests ELISA réalisés sur les pools d'échantillons de sérum, après extrapolation linéaire des valeurs obtenues.
Pour réaliser les tests ELISA, les puits de plaques de microtitration de 96 puits ont été recouverts par incubation avec l'antigène PA83 ou l'antigène LF (List Laboratories) dilués dans du tampon PBS (5µg/ml, 100 µl par puits) pendant une nuit à 4°C. Les sites libres des puits de microplaque ont ensuite été bloqués par incubation avec un volume de 200 µl d'une solution d'albumine sérique bovine (BSA) à 5% dans du tampon PBS, pendant 1 heure à 37°C. Les sérums ont été dilués en série dans un tampon de PBS à 0,1 % de Tween® 20, 1% de BSA, puis incubés dans les plaques (100 µl/puits), pendant 2 heures à 37°C. Les puits des plaques ont été ensuite incubés avec un conjugué IgG anti-souris/phosphatase alcaline ou un conjugué IgG antihumain/ phosphatase alcaline dilué au 1/10 000 (Sigma, Saint Louis, Missouri, Etats- Unis), pendant 1 heure à 37°C. Le substrat phosphate de P-nitrophényle (Sigma) a ensuite été ajouté et les plaques ont été incubées pendant 30 minutes à température ambiante. On a déterminé l'absorbance à 405 nm en utilisant un lecteur automatisé de microplaques (iEMS reader MF, Labsystems, Helsinki, Finlande). On a défini le point de dilution limite, dont la valeur réciproque correspond au titre anticorps du sérum, comme le point pour lequel la valeur du signal était égale au double de la valeur du signal mesurée pour le sérum de souris naïves. Le sérum de souris naïves est utilisé comme témoin négatif.

### Exemple 7 : Etudes de protection passive des rats

Pour les essais *in vivo,* on injecte à des rats Fischer (250 à 300g) (C. River, L'Abresle, France) 40 µg de PA (List biological laboratories, Campbell, CA) et 8 µg de LF par 250g de rat, de la manière décrite dans Ezzel *et al.* (Ezzell *et al.,* 1984), excepté le fait que la veine de la queue est utilisée. 4 animaux sont utilisés par groupe et pour l'évaluation de l'immunoglobuline 35PA83 «6.20», l'immunoglobuline est ajoutée au PA et au LF avant l'injection. On observe les rats 2 fois par jour pendant 10 jours. Tous les essais in vivo présentés dans cette étude sont approuvés par le comité d'éthique local pour l'expérimentation et le soin animal.

### Préparation et utilisation des spores Sterne :

Des spores de *B. anthracis* Sterne (collection Pasteur) sont préparées comme exposé dans Albrecht *et al.* (Albrecht *et al.,* 2007), et gardées congelées (-20°C). Les spores sont dénombrées par comptage de plaque viable après congélation/décongélation et le compte est vérifié quand chaque tube est utilisé dans cette étude. La LD50 de ces spores administrées par voie intraveineuse dans des souris males A/J (Harlan, Gannat, France), âgées de 9 semaines, pesant 20-25g, est établie à 1.10⁴, menant à la mort en 48 à 72 heures, soit proche d'une valeur de 2.10(4) utilisée dans une autre étude (Albrecht *et al.,* 2007).

### Exemple 8 : Prophylaxie par l'immunoglobuline 35PA83 « 6.20 », court traitement avec la tétracycline, ou les 2

Pour les études d'un schéma prophylactique de l'immunoglobuline 35PA83 «6.20» ou par tétracycline seulement, on injecte les immunoglobulines à des groupes de 10 souris A/J, par voie subcutanée, 12 heures avant l'infection (une injection de 5 mg/kg ou de 2 mg/kg). Le challenge est administré comme 10 000 LD₅₀ ou 1.10⁸ spores et les souris sont observées 2 fois par jour pendant 2 semaines, puis 5 fois par semaine pendant 2 semaines additionnelles. Les souris survivantes sont re-testées par infection un mois plus tard, avec la même quantité de spores, et observées pendant un mois additionnel. Pour les études d'un schéma prophylactique impliquant à la fois la tétracycline et l'immunoglobuline 35PA83 «6.20», des groupes de 10 souris sont traités avec la tétracycline comme dans le schéma impliquant la tétracycline seule mais, en plus, l'immunoglobuline 35PA83 «6.20» est injectée 12 heures avant le challenge. Pour les études de protection active, 10 souris sont injectées par voie subcutanée avec 5 µg de PA par souris, dans de l'adjuvant complet de Freund. Un second groupe reçoit la même injection puis, 1 mois plus tard, la réponse immunitaire de ce groupe est stimulée avec la même dose de PA dans de l'adjuvant de Freund incomplet.

### Exemple 9 : Prophylaxie par l'immunoglobuline 35PA83 « 6.20 », court traitement avec la doxycycline, ou les 2

L'étude de la prophylaxie avec la doxycycline, avec ou sans l'immunoglobuline 35PA83 « 6.20 », a été réalisée avec des groupes de dix souris A/J âgées de 10 semaines (Harlan, Gannat, France), auxquelles on a injecté de manière prophylactique l'antibiotique par voie intra péritonéale, à la dose unique quotidienne de 5 mg/kg. La chimioprophylaxie a débuté 12 heures avant l'infection et a été réalisée pendant 7 jours, ce qui représente une réduction de 9/10 de la durée standard qui est de 60 jours.

On a choisi un dosage de doxycycline qui est approximativement le double du dosage humain standard (dosage quotidien de 3 mg/kg pour un humain adulte), et on a montré que des doses plus petites étaient efficaces contre *B. anthracis* (Friedlander et al., 1993, J Infect Dis, Vol. 167 : 1239-1243 ; Kalns et al., 2002, Biochem Biophys Res Commun, Vol. 297 : 506-509). Des doses plus grandes ont été utilisées (Heine et al., 2007, Antimicrob Agents Chemother, Vol. 51 : 1373-1379) ; on a toutefois observé qu'une dose de 50 mg/kg semblait mal tolérée chez les souris A/J, qui présentaient alors un gonflement de l'abdomen et un poil hérissé. Pour complémenter le traitement par la doxycycline avec l'immunoglobuline 35PA83 «6.20», une dose unique de cet anticorps (1 ou 2 mg/kg) a été injectée ou non de manière concomitante à la dernière dose de doxycycline. L'infection a utilisé 1.10⁸ spores injectées par voie intra péritonéale, ce qui représente 10 000 LD₅₀. Les souris ont été observées deux fois par jour pendant les deux premières semaines, puis cinq fois par semaine pendant les deux semaines additionnelles.

### Exemple 10 : Thérapie avec l'immunoglobuline 35PA83 «6.20», ciprofloxacine court traitement ou les 2

Pour les études de schéma thérapeutique, des groupes de 10 souris A/J sont challengées avec une dose de 1000 LD50 ou 1.10⁷ spores. Après 12 heures, l'immunoglobuline 35PA83 «6.20» (voie sous-cutanée, 1 injection de 10 mg/kg) ou la ciprofloxacine (voie subcutanée, 50 mg/kg 2 fois par jours pendant 5 jours) sont injectés séparément ou la ciprofloxacine et l'immunoglobuline 35PA83 «6.20» sont toutes deux injectées le premier jour puis la ciprofloxacine seule est encore injectée pendant 4 jours additionnels.

### Exemple 11 : Thérapie avec l'immunoglobuline 35PA83 «6.20», ciprofloxacine court traitement ou les 2 (autre essai)

Pour les études de traitement curatif, des groupes de 10 souris A/J sont infectées avec une dose de 1000 LD₅₀ ou 1.10⁷ spores. Après 12 heures, les souris ont été traitées avec la ciprofloxacine (par voie sous-cutanée, avec une injection initiale de 25 mg/kg) ou avec I'IgG 35PA83 « 6.20 » (voie subcutanée, 1 injection de 10 mg/kg) séparément ; ou la ciprofloxacine et I'IgG 35PA83 «6.20» sont toutes deux injectées simultanément à deux sites différents. On a aussi testé des délais additionnels de 24 heures et 48 heures avant de débuter le traitement combiné (ciprofloxacine et IgG 35PA83 «6.20»). Après la première administration du traitement, la ciprofloxacine seule (25 mg/kg, deux fois par jour) a été injectée durant les 4,5 jours suivants. La dose de ciprofloxacine a été choisie comme étant approximativement le double de la dose standard humaine (dose quotidienne de 20 mg/kg chez l'homme adulte), cette dose ayant déjà été utilisée efficacement contre B. anthracis (Kalns et al., 2002, Biochem Biophys Res Commun, Vol. 297 : 506-509). La tolérance à cette dose sélectionnée a été favorablement testée chez les souris A/J avant de débuter cette étude. Cette partie de l'étude vise essentiellement à résoudre le problème de la survie à court-terme suivant un traitement différé, et la surveillance a été limitée à la période de 18 jours suivant l'infection.

### Exemple 12 : Comparaison entre les traitements anti-anthrax prophylactiques passifs et actifs

Un traitement anti-anthrax prophylactique passif consiste en un traitement avec l'immunoglobuline 35PA83 «6.20». Un traitement anti-anthrax prophylactique actif consiste en un traitement par immunisation avec l'antigène PA.
Pour comparer l'immuno-protection active et passive, un groupe de dix souris ont été immunisées par voie sous-cutanée avec 5 µg de PA83 dans de l'adjuvant complet de Freund et infectées par voie intra péritonéale avec 10 000 LD₅₀, un mois après. Un autre groupe de dix souris ont été immunisées de manière identique, mais ont reçu une immunisation de rappel quatre semaines après avec 5 µg de PA83 dans de l'adjuvant incomplet de Freund et infectées un mois après la seconde injection. En parallèle, on a évalué la protection passive par l'immunoglobuline 35PA83 «6.20» contre la même infection. Tous les animaux infectés ont été observés pendant un mois, et les résultats des deux types de prophylaxie ont été comparés.

### Exemple 13 : Comparaison entre une immunisation passive et un traitement tardif avec l'IgG 35PA83 «6.20» seule chez des lapins White New Zealand (WNZ) infectés par des spores de 9602

Pour l'étude d'immunisation passive, l'IgG 35PA83 «6.20» est injectée en intraveineuse à 2.5, 1, 0.5 mg/kg dans 3 groupes de 8 lapins WNZ, préalablement anesthésiés avec de l'imalgène 1000 (Merial, Lyon, France). Cinq minutes après, les animaux sont mis en contact avec 25µl d'une suspension de spores de la souche virulente *B.anthracis* 9602, déposée sur chaque narine pour inhalation dans les poumons, et correspondant à 100 LD₅₀.
Pour le traitement tardif, les mêmes conditions expérimentales ont été utilisées, excepté que 2 groupes de 8 animaux reçoivent l'injection d'IgG (2.5 mg/kg) 6h après une mise en contact avec 80 LD₅₀ ou 200 LD₅₀ de spores de *B.anthracis* 9602.
Pour chaque groupe, 4 animaux additionnels sont utilisés dans les mêmes conditions expérimentales, comme contrôles positifs. Toutes les expériences avec la souche de *B. anthracis* 9602 sont réalisées dans un laboratoire de niveau de sécurité 3, et les animaux sont observés 21 jours après la mise en contact.

### Références bibliographiques

Albrecht, M. T., H. Li, E. D. Williamson, C. S. Lebutt, H. C. Flick-Smith, C. P. Quinn, H. Westra, D. Galloway, A. Mateczun, S. Goldman, H. Groen, and L. W. Baillie. 2007. Human monoclonal antibodies against anthrax lethal factor and protective antigen act independently to protect against Bacillus anthracis infection and enhance endogenous immunity to anthrax. Infect Immun.
Andhs-Widhopf, J., C. Rader, P. Steinberger, R. Fuller, and C. F. Barbas, 3rd. 2000. Methods for the generation of chicken monoclonal antibody fragments by phage display. J Immunol Methods 242:159-181.
Andhs-Widhopf, J., P. Steinberger, R. Fuller, C. Rader, and C. F. Barbas, 3rd. 2001. Generation of antibody libraries: PCR amplification and assembly of light - and heavy-chain coding sequences. In C. F. Barbas, 3rd, D. R. Burton, J. K. Scott, and G. J. Silverman (ed.), Phage Display: A Laboratory Manual. Cold Spring Harbor Laboratory Press, New York.
Carter, P., H. Bedouelle, and G. Winter. 1985. Improved oligonucleotide site- directed mutagenesis using M13 vectors. Nucleic Acids Res 13:4431 -43. Ezzell, J. W., B. E. Ivins, and S. H. Leppla. 1984. Immunoelectrophoretic analysis, toxicity, and kinetics of in vitro production of the protective antigen and lethal factor components of Bacillus anthracis toxin. Infect Immun 45:761 -7.
Karlsson, R., A. Michaelsson, and L. Mattsson. 1991. Kinetic analysis of monoclonal antibody-antigen interactions with a new biosensor based analytical System. J Immunol Methods 145:229-40).
Laffly, " Selection of a macaque Fab with framework regions like those in humans, high affinity, and ability to neutralize the protective antigen (PA) of bacillus anthracis by binding to segment of PA between residues 686 and 694 ", Antimicrobial agents and chemotherapy, Aug. 2005, p. 3414-3420.
Little, S. F., S. H. Leppla, and A. M. Friedlander. 1990. Production and characterization of monoclonal antibodies against the lethal factor component of Bacillus anthracis lethal toxin. Infect Immun 58:1606-13.
Schuck, P., and A. P. Minton. 1996. Analysis of mass transport-limited binding kinetics in evanescent wave biosensors. Anal Biochem 240:262-72.

## Revendications

1. Immunoglobuline de classe G dirigée contre l'antigène protecteur de la toxine du charbon, dans laquelle :
- chacune des chaînes lourdes comprend, ou consiste en, une séquence d'acides aminés représentée par SEQ ID NO : 5, et
- chacune des chaînes légères comprend, ou consiste en, une séquence d'acides aminés représentée par SEQ ID NO : 6.

2. Immunoglobuline selon la revendication 1, dans laquelle :
- la région constante de ladite chaîne lourde comprend une séquence d'acides aminés représentée par la séquence SEQ ID NO : 3, et
- la région constante de chaîne légère comprend une séquence d'acides aminés représentée par la séquence SEQ ID NO : 4.

3. Immunoglobuline selon l'une quelconque des revendications précédentes, dans laquelle :
- chacune des chaînes lourdes comprend une séquence d'acides aminés représentée par SEQ ID NO : 7, et
- chacune des chaînes légères comprend une séquence d'acides aminés représentée par SEQ ID NO : 8.

4. Immunoglobuline selon l'une quelconque des revendications précédentes, possédant sur son site de glycosylation Asn297 des N-glycannes dont le taux de fucosylation est inférieur à 65%, de préférence inférieur à 50%, de préférence encore inférieur à 40%.

5. Immunoglobuline selon la revendication 4, présentant une teneur supérieure à 60 % pour les formes G0+G1+G0F+G1F, les formes G0F+G1F étant inférieures à 50 %.

6. Immunoglobuline selon la revendication 5, présentant une teneur supérieure à 60 % pour les formes G0+G1+G0F+G1F, la teneur en fucose étant inférieure à 65 %.

7. Immunoglobuline selon la revendication 5, présentant une teneur inférieure à 40% pour les formes G1F+GOF.

8. Polynucléotide comprenant au moins une séquence nucléotidique codant la chaîne lourde et une séquence codant la chaîne légère d'une immunoglobuline telle que définie selon l'une quelconque des revendications 1 à 7.

9. Vecteur comprenant au moins un polynucléotide selon la revendication 8.

10. Cellule hôte comprenant un vecteur selon la revendication 9.

11. Composition, de préférence pharmaceutique, comprenant au moins une immunoglobuline, de préférence humaine, selon l'une quelconque des revendications 1 à 7.

12. Immunoglobuline, de préférence humaine, selon l'une quelconque des revendications 1 à 7, pour son utilisation comme médicament.

13. Immunoglobuline, de préférence humaine, selon l'une quelconque des revendications 1 à 7, pour son utilisation dans le traitement ou la prévention de pathologies liées à la toxine du charbon.

14. Méthode pour la détection *in vitro* d'une toxine du charbon comprenant l'antigène protecteur dans un échantillon biologique, comprenant :
- la mise en contact de l'échantillon avec au moins une immunoglobuline selon l'une quelconque des revendications 1 à 7, et
- la détection de la liaison de ladite immunoglobuline comme indicateur de la présence de ladite toxine du charbon.

15. Kit pour la détection d'une toxine du charbon comprenant l'antigène protecteur, ledit kit comprenant :
- un récipient comprenant au moins une immunoglobuline selon l'une quelconque des revendications 1 à 7 marquée, et
- un récipient comprenant des moyens de détection de ladite immunoglobuline marquée.

16. Immunoconjugué comprenant une immunoglobuline selon l'une quelconque des revendications 1 à 7 liée à un agent thérapeutique.

## Patentansprüche

1. Immunglobulin der Klasse G, gerichtet gegen das Schutz-Antigen des Anthrax-Toxins, wobei:
- jede der schweren Ketten eine Aminosäurensequenz umfasst oder daraus besteht, dargestellt durch SEQ ID NO : 5, und
- jede der leichten Ketten eine Aminosäurensequenz umfasst oder daraus besteht, dargestellt durch SEQ ID NO : 6.

2. Immunglobulin nach Anspruch 1, wobei:
- die konstante Region der schweren Kette eine Aminosäurensequenz umfasst, dargestellt durch die Sequenz SEQ ID NO : 3, und
- die konstante Region der leichten Kette eine Aminosäurensequenz umfasst, dargestellt durch die Sequenz SEQ ID NO : 4.

3. Immunglobulin nach einem der vorangehenden Ansprüche, wobei:
- jede der schweren Ketten eine Aminosäurensequenz umfasst, dargestellt durch SEQ ID NO : 7, und
- jede der leichten Ketten eine Aminosäurensequenz umfasst, dargestellt durch SEQ ID NO : 8.

4. Immunglobulin nach einem der vorangehenden Ansprüche, das auf seiner Glycosylierungsstelle Asn297 N-Glycane besitzt, deren Fucosylierungsgrad unter 65 %, vorzugsweise unter 50 %, noch vorzugsweiser unter 40 % beträgt.

5. Immunglobulin nach Anspruch 4, aufweisend einen Gehalt von über 60 % für die Formen G0+G1+G0F+G1F, wobei die Formen G0F+G1F unter 50 % sind.

6. Immunglobulin nach Anspruch 5, aufweisend einen Gehalt von über 60 % für die Formen G0+G1+G0F+G1F, wobei der Fucosegehalt unter 65 % ist.

7. Immunglobulin nach Anspruch 5, aufweisend einen Gehalt von unter 40 % für die Formen G1F+GOF.

8. Polynukleotid, umfassend mindestens eine Nukleotidsequenz, welche die schwere Kette codiert, und eine Sequenz, welche die leichte Kette codiert, von einem Immunglobulin nach einem der Ansprüche 1 bis 7.

9. Vektor, umfassend mindestens ein Polynukleotid nach Anspruch 8.

10. Wirtszelle, umfassend einen Vektor nach Anspruch 9.

11. Vorzugsweise pharmazeutische Zusammensetzung, umfassend mindestens ein vorzugsweises humanes Immunglobulin nach einem der Ansprüche 1 bis 7.

12. Vorzugsweises humanes Immunglobulin nach einem der Ansprüche 1 bis 7 für seine Verwendung als Arzneimittel.

13. Vorzugsweise humanes Immunglobulin nach einem der Ansprüche 1 bis 7 für seine Verwendung bei der Behandlung oder der Vorbeugung von Erkrankungen, die mit dem Anthrax-Toxin verbunden sind.

14. Methode für den in vitro-Nachweis eines Anthrax-Toxins, umfassend das Schutzantigen in einer biologischen Probe, umfassend:
- das Inkontaktversetzen der Probe mit mindestens einem Immunglobulin nach einem der Ansprüche 1 bis 7, und
- das Nachweisen der Verbindung des Immunglobulins als Indikator des Vorhandenseins des Anthrax-Toxins.

15. Kit für den Nachweis eines Anthrax-Toxins, umfassend das Schutzantigen, wobei das Kit umfasst:
- einen Behälter, umfassend mindestens ein markiertes Immunglobulin nach einem der Ansprüche 1 bis 7, und
- einen Behälter, umfassend Nachweismittel des markierten Immunglobulins.

16. Immunkonjugat, umfassend ein Immunglobulin nach einem der Ansprüche 1 bis 7, verbunden mit einem therapeutischen Wirkstoff.

## Claims

1. A class-G immunoglobulin directed against the anthrax toxin protective antigen, wherein:
- each of the heavy chains comprises, or consists of, an amino acid sequence represented by SEQ ID NO:5, and
- each of the light chains comprises, or consists of, an amino acid sequence represented by SEQ ID NO:6.

2. The immunoglobulin according to claim 1, wherein:
- the constant region of said heavy chain comprises an amino acid sequence represented by sequence SEQ ID NO:3, and
- the light chain constant region comprises an amino acid sequence represented by sequence SEQ ID NO:4.

3. The immunoglobulin according to any one of the preceding claims, wherein:
- each of the heavy chains comprises an amino acid sequence represented by SEQ ID NO:7, and
- each of the light chains comprises an amino acid sequence represented by SEQ ID NO:8.

4. The immunoglobulin according to any one of the preceding claims, having on its Asn297 glycosylation site N-glycans having a degree of fucosylation lower than 65%, preferably lower than 50%, more preferably lower than 40%.

5. The immunoglobulin according to claim 4, having a content of G0+G1+G0F+G1F forms greater than 60%, wherein G0F+G1F forms are less than 50%.

6. The immunoglobulin according to claim 5, having a content of G0+G1+G0F+G1F forms greater than 60%, and wherein fucose content is lower than 65%.

7. The immunoglobulin according to claim 5, having a content of G1F+G0F forms lower than 40%.

8. A polynucleotide comprising at least one nucleotide sequence encoding the heavy chain and a sequence encoding the light chain of an immunoglobulin as defined in any one of claim 1 to 7.

9. A vector comprising at least one polynucleotide according to claim 8.

10. A host cell comprising a vector according to claim 9.

11. A composition, preferably a pharmaceutical composition, comprising at least one immunoglobulin, preferably human immunoglobulin, according to any one of claim 1 to 7.

12. The immunoglobulin, preferably a human immunoglobulin, according to any one of claims 1 to 7, for use as a medicine.

13. The immunoglobulin, preferably a human immunoglobulin, according to any one of claims 1 to 7, for use in the treatment or the prevention of pathologies related to the anthrax toxin.

14. A method for in vitro detection of an anthrax toxin comprising the protective antigen in a biological sample, comprising:
- contacting the sample with at least one immunoglobulin according to any one of claims 1 to 7, and
- detecting the binding of said immunoglobulin as an indicator of the presence of said anthrax toxin.

15. A kit for detecting an anthrax toxin comprising the protective antigen, said kit comprising:
- a container comprising at least one immunoglobulin according to any one of claims 1 to 7, wherein said immunoglobulin is labeled, and
- a container comprising means for detecting said labeled immunoglobulin.

16. An immunoconjugate comprising an immunoglobulin according to any one of claims 1 to 7, bound to a therapeutic agent.


### SEQUENCE LISTING

<110> LFB et al
<120> IMMUNOGLOBULINE ANTI-TOXINE DU CHARBON
<130> BFF130656 CEB
<160> 20
<170> PatentIn version 3.5
<210> 1
   <211> 125
   <212> PRT
   <213> Artificial Sequence
<220>
   <223> VH
<400> 1
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Gly Gly
               20                  25                  30
   Tyr Tyr Trp Ser Trp Ile Arg Gln Ser Pro Gly Lys Gly Leu Glu Trp
           35                  40                  45
   Ile Gly Leu Ile Tyr Gly Ser Thr Ala Asp Thr Arg Tyr Asn Pro Ser
       50                  55                  60
   Leu Lys Gly Arg Val Thr Ile Ser Lys Asp Thr Ser Lys Asn Gln Leu
   65                  70                  75                  80
   Ser Leu Gln Leu Arg Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Ala Val Val Thr Val Ser Ser
           115                 120                 125
<210> 2
   <211> 107
   <212> PRT
   <213> Artificial Sequence
<220>
   <223> VL
<400> 2
   Ala Ile Gln Leu Thr Gln Ser Pro Ser Ser Leu Ser Ala Tyr Val Gly
   1               5                   10                  15
   Asp Lys Val Thr Ile Thr Cys His Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Leu Ser Gly Val Pro Ser Arg Phe Ser Gly
       50                  55                  60
   Ser Gly Ser Gly Thr Asp Tyr Thr Leu Thr Ile Ser Ser Leu Gln Ser
   65                  70                  75                  80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys
               100                 105
<210> 3
   <211> 330
   <212> PRT
   <213> Artificial Sequence
<220>
   <223> région constante de la chaîne lourde
<400> 3
<210> 4
   <211> 107
   <212> PRT
   <213> Artificial Sequence
<220>
   <223> Région constante de la chaîne légère
<400> 4
<210> 5
   <211> 455
   <212> **PRT**
   <213> Artificial Sequence
<220>
   <223> chaîne lourde sans peptide signal
<400> 5
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Gly Gly
               20                  25                  30
   Tyr Tyr Trp Ser Trp Ile Arg Gln Ser Pro Gly Lys Gly Leu Glu Trp
           35                  40                  45
   Ile Gly Leu Ile Tyr Gly Ser Thr Ala Asp Thr Arg Tyr Asn Pro Ser
       50                  55                  60
   Leu Lys Gly Arg Val Thr Ile Ser Lys Asp Thr Ser Lys Asn Gln Leu
   65                  70                  75                  80
   Ser Leu Gln Leu Arg Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Ala Val Val Thr Val Ser Ser Ala Ser Thr
           115                 120                 125
   Lys Gly Pro Ser Val Phe Pro Leu Ala Pro Ser Ser Lys Ser Thr Ser
       130                 135                 140
   Gly Gly Thr Ala Ala Leu Gly Cys Leu Val Lys Asp Tyr Phe Pro Glu
   145                 150                 155                 160
   Pro Val Thr Val Ser Trp Asn Ser Gly Ala Leu Thr Ser Gly Val His
                   165                 170                 175
   Thr Phe Pro Ala Val Leu Gln Ser Ser Gly Leu Tyr Ser Leu Ser Ser
               180                 185                 190
   Val Val Thr Val Pro Ser Ser Ser Leu Gly Thr Gln Thr Tyr Ile Cys
           195                 200                 205
   Asn Val Asn His Lys Pro Ser Asn Thr Lys Val Asp Lys Lys Val Glu
       210                 215                 220
   Pro Lys Ser Cys Asp Lys Thr His Thr Cys Pro Pro Cys Pro Ala Pro
   225                 230                 235                 240
   Glu Leu Leu Gly Gly Pro Ser Val Phe Leu Phe Pro Pro Lys Pro Lys
                   245                 250                 255
   Asp Thr Leu Met Ile Ser Arg Thr Pro Glu Val Thr Cys Val Val Val
               260                 265                 270
   Asp Val Ser His Glu Asp Pro Glu Val Lys Phe Asn Trp Tyr Val Asp
           275                 280                 285
   Gly Val Glu Val His Asn Ala Lys Thr Lys Pro Arg Glu Glu Gln Tyr
       290                 295                 300
   Asn Ser Thr Tyr Arg Val Val Ser Val Leu Thr Val Leu His Gln Asp
   305                 310                 315                 320
   Trp Leu Asn Gly Lys Glu Tyr Lys Cys Lys Val Ser Asn Lys Ala Leu
                   325                 330                 335
   Pro Ala Pro Ile Glu Lys Thr Ile Ser Lys Ala Lys Gly Gln Pro Arg
               340                 345                 350
   Glu Pro Gln Val Tyr Thr Leu Pro Pro Ser Arg Asp Glu Leu Thr Lys
           355                 360                 365
   Asn Gln Val Ser Leu Thr Cys Leu Val Lys Gly Phe Tyr Pro Ser Asp
       370                 375                 380
   Ile Ala Val Glu Trp Glu Ser Asn Gly Gln Pro Glu Asn Asn Tyr Lys
   385                 390                 395                 400
   Thr Thr Pro Pro Val Leu Asp Ser Asp Gly Ser Phe Phe Leu Tyr Ser
                   405                 410                 415
   Lys Leu Thr Val Asp Lys Ser Arg Trp Gln Gln Gly Asn Val Phe Ser
               420                 425                 430
   Cys Ser Val Met His Glu Ala Leu His Asn His Tyr Thr Gln Lys Ser
           435                 440                 445
   Leu Ser Leu Ser Pro Gly Lys
       450                 455
<210> 6
   <211> 214
   <212> PRT
   <213> Artificial Sequence
<220>
   <223> chaîne légère sans peptide signal
<400> 6
   Ala Ile Gln Leu Thr Gln Ser Pro Ser Ser Leu Ser Ala Tyr Val Gly
   1               5                   10                  15
   Asp Lys Val Thr Ile Thr Cys His Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Leu Ser Gly Val Pro Ser Arg Phe Ser Gly
       50                  55                  60
   Ser Gly Ser Gly Thr Asp Tyr Thr Leu Thr Ile Ser Ser Leu Gln Ser
   65                  70                  75                  80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 7
   <211> 473
   <212> PRT
   <213> Artificial Sequence
<220>
   <223> chaîne lourde avec peptide signal
<400> 7
   Met Arg Trp Ser Trp Ile Phe Leu Leu Leu Leu Ser Ile Thr Ser Ala
   1               5                   10                  15
   Asn Ala Gln Val Gln Leu Gln Glu Ser Gly Pro Gly Leu Leu Lys Pro
               20                  25                  30
   Ser Glu Thr Leu Ser Leu Thr Cys Ala Val Ser Gly Asp Ser Ile Ser
           35                  40                  45
   Gly Gly Tyr Tyr Trp Ser Trp Ile Arg Gln Ser Pro Gly Lys Gly Leu
       50                  55                  60
   Glu Trp Ile Gly Leu Ile Tyr Gly Ser Thr Ala Asp Thr Arg Tyr Asn
   65                  70                  75                  80
   Pro Ser Leu Lys Gly Arg Val Thr Ile Ser Lys Asp Thr Ser Lys Asn
                   85                  90                  95
   Gln Leu Ser Leu Gln Leu Arg Ser Val Thr Ala Ala Asp Thr Ala Val
               100                 105                 110
   Tyr Tyr Cys Ala Arg Ser Gly Tyr Asn Phe Trp Ser Gly Glu Tyr Tyr
           115                 120                 125
   Gly Leu Asp Ser Trp Gly Gln Gly Ala Val Val Thr Val Ser Ser Ala
       130                 135                 140
   Ser Thr Lys Gly Pro Ser Val Phe Pro Leu Ala Pro Ser Ser Lys Ser
   145                 150                 155                 160
   Thr Ser Gly Gly Thr Ala Ala Leu Gly Cys Leu Val Lys Asp Tyr Phe
                   165                 170                 175
   Pro Glu Pro Val Thr Val Ser Trp Asn Ser Gly Ala Leu Thr Ser Gly
               180                 185                 190
   Val His Thr Phe Pro Ala Val Leu Gln Ser Ser Gly Leu Tyr Ser Leu
           195                 200                 205
   Ser Ser Val Val Thr Val Pro Ser Ser Ser Leu Gly Thr Gln Thr Tyr
       210                 215                 220
   Ile Cys Asn Val Asn His Lys Pro Ser Asn Thr Lys Val Asp Lys Lys
   225                 230                 235                 240
   Val Glu Pro Lys Ser Cys Asp Lys Thr His Thr Cys Pro Pro Cys Pro
                   245                 250                 255
   Ala Pro Glu Leu Leu Gly Gly Pro Ser Val Phe Leu Phe Pro Pro Lys
               260                 265                 270
   Pro Lys Asp Thr Leu Met Ile Ser Arg Thr Pro Glu Val Thr Cys Val
           275                 280                 285
   Val Val Asp Val Ser His Glu Asp Pro Glu Val Lys Phe Asn Trp Tyr
       290                 295                 300
   Val Asp Gly Val Glu Val His Asn Ala Lys Thr Lys Pro Arg Glu Glu
   305                 310                 315                 320
   Gln Tyr Asn Ser Thr Tyr Arg Val Val Ser Val Leu Thr Val Leu His
   325 330 335
   Gln Asp Trp Leu Asn Gly Lys Glu Tyr Lys Cys Lys Val Ser Asn Lys
   340 345 350
   Ala Leu Pro Ala Pro Ile Glu Lys Thr Ile Ser Lys Ala Lys Gly Gln
   355 360 365
   Pro Arg Glu Pro Gln Val Tyr Thr Leu Pro Pro Ser Arg Asp Glu Leu
   370 375 380
   Thr Lys Asn Gln Val Ser Leu Thr Cys Leu Val Lys Gly Phe Tyr Pro
   385 390 395 400
   Ser Asp Ile Ala Val Glu Trp Glu Ser Asn Gly Gln Pro Glu Asn Asn
   405 410 415
   Tyr Lys Thr Thr Pro Pro Val Leu Asp Ser Asp Gly Ser Phe Phe Leu
   420 425 430
   Tyr Ser Lys Leu Thr Val Asp Lys Ser Arg Trp Gln Gln Gly Asn Val
   435 440 445
   Phe Ser Cys Ser Val Met His Glu Ala Leu His Asn His Tyr Thr Gln
   450 455 460
   Lys Ser Leu Ser Leu Ser Pro Gly Lys
   465 470
<210> 8
   <211> 232
   <212> PRT
   <213> Artificial Sequence
<220>
   <223> chaîne légère avec peptide signal
<400> 8
   Met Arg Trp Ser Trp Ile Phe Leu Leu Leu Leu Ser Ile Thr Ser Ala
   1 5 10 15
   Asn Ala Ala Ile Gln Leu Thr Gln Ser Pro Ser Ser Leu Ser Ala Tyr
   20 25 30
   Val Gly Asp Lys Val Thr Ile Thr Cys His Ala Ser Gln Gly Ile Asn
   35 40 45
   Ser Trp Leu Ala Trp Tyr Gln Gln Lys Pro Gly Lys Ala Pro Lys Leu
       50                  55                  60
   Leu Ile Tyr Lys Ala Ser Ser Leu Leu Ser Gly Val Pro Ser Arg Phe
   65                  70                  75                  80
   Ser Gly Ser Gly Ser Gly Thr Asp Tyr Thr Leu Thr Ile Ser Ser Leu
                   85                  90                  95
   Gln Ser Glu Asp Phe Ala Ser Tyr Tyr Cys Leu Gln Tyr Asp Ser Ala
               100                 105                 110
   Pro Leu Ala Phe Gly Pro Gly Thr Lys Leu Asp Ile Lys Arg Thr Val
           115                 120                 125
   Ala Ala Pro Ser Val Phe Ile Phe Pro Pro Ser Asp Glu Gln Leu Lys
       130                 135                 140
   Ser Gly Thr Ala Ser Val Val Cys Leu Leu Asn Asn Phe Tyr Pro Arg
   145                 150                 155                 160
   Glu Ala Lys Val Gln Trp Lys Val Asp Asn Ala Leu Gln Ser Gly Asn
                   165                 170                 175
   Ser Gln Glu Ser Val Thr Glu Gln Asp Ser Lys Asp Ser Thr Tyr Ser
               180                 185                 190
   Leu Ser Ser Thr Leu Thr Leu Ser Lys Ala Asp Tyr Glu Lys His Lys
           195                 200                 205
   Val Tyr Ala Cys Glu Val Thr His Gln Gly Leu Ser Ser Pro Val Thr
       210                 215                 220
   Lys Ser Phe Asn Arg Gly Glu Cys
   225                 230
<210> 9
   <211> 375
   <212> DNA
   <213> Artificial Sequence
<220>
   <223> Séquence nucléotidique codant la partie variable de la chaîne lourde
<400> 9
   caggtgcagc tgcaggaatc tggccctggc ctgctgaagc ccagcgagac actgtctctg      60
   acctgcgccg tgtccggcga ctctatcagc ggcggctact actggtcttg gatcaggcag     120
   agccccggca agggcctgga atggatcggc ctgatctacg gcagcaccgc cgacaccaga    180
   tacaacccca gcctgaaggg cagagtgacc atcagcaagg acaccagcaa gaaccagctg    240
   tctctgcagc tgagaagcgt gaccgctgcc gacaccgccg tgtactactg tgccagaagc    300
   ggctacaact tttggagcgg cgagtactac ggcctggact cttggggaca gggcgctgtc    360
   gtgacagtgt ccagc                                                      375
<210> 10
   <211> 321
   <212> DNA
   <213> Artificial Sequence
<220>
   <223> Séquence nucléotidique codant la partie variable de la chaîne légère
<400> 10
   gccatccagc tgacccagag ccctagctct ctgagcgcct acgtgggcga caaagtgacc     60
   atcacctgtc acgccagcca gggcatcaac agctggctgg cctggtatca gcagaagccc    120
   ggcaaggccc ccaagctgct gatctacaag gccagcagcc tgctgagcgg cgtgcccagc    180
   agattcagcg gctctggctc tggcaccgac tacaccctga ccatcagctc cctgcagagc    240
   gaggacttcg ccagctacta ctgcctgcag tacgacagcg cccctctggc cttcggccct    300
   ggaacaaagc tggacatcaa g                                              321
<210> 11
   <211> 990
   <212> DNA
   <213> Artificial Sequence
<220>
   <223> Séquence nucléotidique codant la partie constante de la chaîne lourde
<400> 11
   gcctctacca agggcccaag cgtgttccct ctggccccta gcagcaagtc tacctctggc        60
   ggaacagccg ccctgggctg cctcgtgaag gactactttc ccgagcccgt gaccgtgtcc       120
   tggaactctg gcgctctgac aagcggcgtg cacaccttcc ctgccgtgct gcagtctagc       180
   ggcctgtaca gcctgagcag cgtcgtgact gtgccctcta gctctctggg cacccagacc       240
   tacatctgca acgtgaacca caagcccagc aacaccaagg tggacaagaa ggtggaaccc       300
   aagagctgcg acaagaccca cacctgtcct ccctgtcctg cccctgaact gctgggcgga       360
   ccttccgtgt tcctgttccc cccaaagcct aaggacaccc tgatgatcag caggaccccc       420
   gaagtgacct gcgtggtggt ggatgtgtcc cacgaggacc ctgaagtgaa gttcaattgg       480
   tacgtggacg gcgtggaagt gcacaacgcc aagaccaagc ctagagagga acagtacaac       540
   agcacctaca gggtggtgtc tgtgctgaca gtgctgcacc aggactggct gaacggcaaa       600
   gagtacaagt gcaaggtgtc caacaaggcc ctgccagccc ccatcgaaaa gaccatctcc       660
   aaggccaagg gacagcctcg cgagccccag gtgtacacac tgcctcccag cagggacgag       720
   ctgacaaaga atcaggtgtc cctgacctgt ctcgtgaaag gcttctaccc cagcgacatt       780
   gccgtggaat gggagagcaa cggccagccc gagaacaact acaagaccac cccccctgtg       840
   ctggacagcg acggctcatt cttcctgtac tccaagctga ccgtggacaa gtccaggtgg       900
   cagcagggca acgtgttcag ctgctccgtg atgcacgagg ccctgcacaa ccactacacc       960
   cagaagtccc tgagcctgag ccctggcaag                                        990
<210> 12
   <211> 321
   <212> DNA
   <213> Artificial Sequence
<220>
   <223> Séquence nucléotidique codant la partie constante de la chaîne légère
<400> 12
   aggaccgtgg ccgcaccaag tgtctttatc ttcccaccca gcgacgagca gctgaagtcc        60
   ggcacagctt ccgtcgtgtg cctgctgaac aacttctacc ctagggaagc caaggtgcag       120
   tggaaggtgg acaacgccct gcagtccggc aactcccagg aaagcgtgac cgagcaggac       180
   agcaaggact ccacctacag cctgagcagc accctgacac tgagcaaggc cgactacgag       240
   aaacataagg tgtacgcctg cgaagtgacc caccagggcc tgtctagccc cgtgaccaag       300
   agcttcaaca ggggcgagtg c                                                 321
<210> 13
   <211> 1365
   <212> DNA
   <213> Artificial Sequence
<220>
   <223> Séquence nucléotidique codant la chaîne lourde
<400> 13
   caggtgcagc tgcaggaatc tggccctggc ctgctgaagc ccagcgagac actgtctctg      60
   acctgcgccg tgtccggcga ctctatcagc ggcggctact actggtcttg gatcaggcag     120
   agccccggca agggcctgga atggatcggc ctgatctacg gcagcaccgc cgacaccaga     180
   tacaacccca gcctgaaggg cagagtgacc atcagcaagg acaccagcaa gaaccagctg     240
   tctctgcagc tgagaagcgt gaccgctgcc gacaccgccg tgtactactg tgccagaagc     300
   ggctacaact tttggagcgg cgagtactac ggcctggact cttggggaca gggcgctgtc     360
   gtgacagtgt ccagcgcctc taccaagggc ccaagcgtgt tccctctggc ccctagcagc     420
   aagtctacct ctggcggaac agccgccctg ggctgcctcg tgaaggacta ctttcccgag     480
   cccgtgaccg tgtcctggaa ctctggcgct ctgacaagcg gcgtgcacac cttccctgcc     540
   gtgctgcagt ctagcggcct gtacagcctg agcagcgtcg tgactgtgcc ctctagctct     600
   ctgggcaccc agacctacat ctgcaacgtg aaccacaagc ccagcaacac caaggtggac     660
   aagaaggtgg aacccaagag ctgcgacaag acccacacct gtcctccctg tcctgcccct     720
   gaactgctgg gcggaccttc cgtgttcctg ttccccccaa agcctaagga caccctgatg     780
   atcagcagga cccccgaagt gacctgcgtg gtggtggatg tgtcccacga ggaccctgaa     840
   gtgaagttca attggtacgt ggacggcgtg gaagtgcaca acgccaagac caagcctaga     900
   gaggaacagt acaacagcac ctacagggtg gtgtctgtgc tgacagtgct gcaccaggac     960
   tggctgaacg gcaaagagta caagtgcaag gtgtccaaca aggccctgcc agcccccatc    1020
   gaaaagacca tctccaaggc caagggacag cctcgcgagc cccaggtgta cacactgcct    1080
   cccagcaggg acgagctgac aaagaatcag gtgtccctga cctgtctcgt gaaaggcttc    1140
   taccccagcg acattgccgt ggaatgggag agcaacggcc agcccgagaa caactacaag    1200
   accacccccc ctgtgctgga cagcgacggc tcattcttcc tgtactccaa gctgaccgtg    1260
   gacaagtcca ggtggcagca gggcaacgtg ttcagctgct ccgtgatgca cgaggccctg    1320
   cacaaccact acacccagaa gtccctgagc ctgagccctg gcaag                    1365
<210> 14
   <211> 642
   <212> DNA
   <213> Artificial Sequence
<220>
   <223> Séquence nucléotidique codant la chaîne légère
<400> 14
   gccatccagc tgacccagag ccctagctct ctgagcgcct acgtgggcga caaagtgacc        60
   atcacctgtc acgccagcca gggcatcaac agctggctgg cctggtatca gcagaagccc       120
   ggcaaggccc ccaagctgct gatctacaag gccagcagcc tgctgagcgg cgtgcccagc       180
   agattcagcg gctctggctc tggcaccgac tacaccctga ccatcagctc cctgcagagc       240
   gaggacttcg ccagctacta ctgcctgcag tacgacagcg cccctctggc cttcggccct       300
   ggaacaaagc tggacatcaa gaggaccgtg gccgcaccaa gtgtctttat cttcccaccc       360
   agcgacgagc agctgaagtc cggcacagct tccgtcgtgt gcctgctgaa caacttctac       420
   cctagggaag ccaaggtgca gtggaaggtg gacaacgccc tgcagtccgg caactcccag       480
   gaaagcgtga ccgagcagga cagcaaggac tccacctaca gcctgagcag caccctgaca       540
   ctgagcaagg ccgactacga gaaacataag gtgtacgcct gcgaagtgac ccaccagggc       600
   ctgtctagcc ccgtgaccaa gagcttcaac aggggcgagt gc                          642
<210> 15
   <211> 1419
   <212> DNA
   <213> Artificial Sequence
<220>
   <223> Séquence nucléotidique codant la chaîne lourde comprenant un peptide signal en N-terminal
<400> 15
   atgcgatggt cctggatctt cctgctgctg ctgagcatca ccagcgccaa cgctcaggtg 60
   cagctgcagg aatctggccc tggcctgctg aagcccagcg agacactgtc tctgacctgc 120
   gccgtgtccg gcgactctat cagcggcggc tactactggt cttggatcag gcagagcccc 180
   ggcaagggcc tggaatggat cggcctgatc tacggcagca ccgccgacac cagatacaac 240
   cccagcctga agggcagagt gaccatcagc aaggacacca gcaagaacca gctgtctctg 300
   cagctgagaa gcgtgaccgc tgccgacacc gccgtgtact actgtgccag aagcggctac 360
   aacttttgga gcggcgagta ctacggcctg gactcttggg gacagggcgc tgtcgtgaca 420
   gtgtccagcg cctctaccaa gggcccaagc gtgttccctc tggcccctag cagcaagtct 480
   acctctggcg gaacagccgc cctgggctgc ctcgtgaagg actactttcc cgagcccgtg 540
   accgtgtcct ggaactctgg cgctctgaca agcggcgtgc acaccttccc tgccgtgctg 600
   cagtctagcg gcctgtacag cctgagcagc gtcgtgactg tgccctctag ctctctgggc 660
   acccagacct acatctgcaa cgtgaaccac aagcccagca acaccaaggt ggacaagaag 720
   gtggaaccca agagctgcga caagacccac acctgtcctc cctgtcctgc ccctgaactg 780
   ctgggcggac cttccgtgtt cctgttcccc ccaaagccta aggacaccct gatgatcagc 840
   aggacccccg aagtgacctg cgtggtggtg gatgtgtccc acgaggaccc tgaagtgaag 900
   ttcaattggt acgtggacgg cgtggaagtg cacaacgcca agaccaagcc tagagaggaa 960
   cagtacaaca gcacctacag ggtggtgtct gtgctgacag tgctgcacca ggactggctg 1020
   aacggcaaag agtacaagtg caaggtgtcc aacaaggccc tgccagcccc catcgaaaag 1080
   accatctcca aggccaaggg acagcctcgc gagccccagg tgtacacact gcctcccagc 1140
   agggacgagc tgacaaagaa tcaggtgtcc ctgacctgtc tcgtgaaagg cttctacccc 1200
   agcgacattg ccgtggaatg ggagagcaac ggccagcccg agaacaacta caagaccacc 1260
   ccccctgtgc tggacagcga cggctcattc ttcctgtact ccaagctgac cgtggacaag 1320
   tccaggtggc agcagggcaa cgtgttcagc tgctccgtga tgcacgaggc cctgcacaac 1380
   cactacaccc agaagtccct gagcctgagc cctggcaag 1419
<210> 16
   <211> 696
   <212> DNA
   <213> Artificial Sequence
<220>
   <223> Séquence nucléotidique codant la chaîne légère comprenant un peptide signal en N-terminal
<400> 16
   atgcgatggt cctggatctt cctgctgctg ctgagcatca ccagcgccaa cgccgccatc        60
   cagctgaccc agagccctag ctctctgagc gcctacgtgg gcgacaaagt gaccatcacc       120
   tgtcacgcca gccagggcat caacagctgg ctggcctggt atcagcagaa gcccggcaag       180
   gcccccaagc tgctgatcta caaggccagc agcctgctga gcggcgtgcc cagcagattc       240
   agcggctctg gctctggcac cgactacacc ctgaccatca gctccctgca gagcgaggac       300
   ttcgccagct actactgcct gcagtacgac agcgcccctc tggccttcgg ccctggaaca       360
   aagctggaca tcaagaggac cgtggccgca ccaagtgtct ttatcttccc acccagcgac       420
   gagcagctga agtccggcac agcttccgtc gtgtgcctgc tgaacaactt ctaccctagg       480
   gaagccaagg tgcagtggaa ggtggacaac gccctgcagt ccggcaactc ccaggaaagc       540
   gtgaccgagc aggacagcaa ggactccacc tacagcctga gcagcaccct gacactgagc       600
   aaggccgact acgagaaaca taaggtgtac gcctgcgaag tgacccacca gggcctgtct       660
   agccccgtga ccaagagctt caacaggggc gagtgc                                 696
<210> 17
   <211> 1448
   <212> DNA
   <213> Artificial Sequence
<220>
   <223> Séquence nucléotidique codant la chaîne lourde telle que synthétisée
<400> 17
   gctagcgccg ccaccatgcg atggtcctgg atcttcctgc tgctgctgag catcaccagc        60
   gccaacgctc aggtgcagct gcaggaatct ggccctggcc tgctgaagcc cagcgagaca       120
   ctgtctctga cctgcgccgt gtccggcgac tctatcagcg gcggctacta ctggtcttgg       180
   atcaggcaga gccccggcaa gggcctggaa tggatcggcc tgatctacgg cagcaccgcc       240
   gacaccagat acaaccccag cctgaagggc agagtgacca tcagcaagga caccagcaag       300
   aaccagctgt ctctgcagct gagaagcgtg accgctgccg acaccgccgt gtactactgt       360
   gccagaagcg gctacaactt ttggagcggc gagtactacg gcctggactc ttggggacag       420
   ggcgctgtcg tgacagtgtc cagcgcctct accaagggcc caagcgtgtt ccctctggcc       480
   cctagcagca agtctacctc tggcggaaca gccgccctgg gctgcctcgt gaaggactac       540
   tttcccgagc ccgtgaccgt gtcctggaac tctggcgctc tgacaagcgg cgtgcacacc       600
   ttccctgccg tgctgcagtc tagcggcctg tacagcctga gcagcgtcgt gactgtgccc       660
   tctagctctc tgggcaccca gacctacatc tgcaacgtga accacaagcc cagcaacacc    720
   aaggtggaca agaaggtgga acccaagagc tgcgacaaga cccacacctg tcctccctgt    780
   cctgcccctg aactgctggg cggaccttcc gtgttcctgt tccccccaaa gcctaaggac     840
   accctgatga tcagcaggac ccccgaagtg acctgcgtgg tggtggatgt gtcccacgag    900
   gaccctgaag tgaagttcaa ttggtacgtg gacggcgtgg aagtgcacaa cgccaagacc    960
   aagcctagag aggaacagta caacagcacc tacagggtgg tgtctgtgct gacagtgctg   1020
   caccaggact ggctgaacgg caaagagtac aagtgcaagg tgtccaacaa ggccctgcca   1080
   gcccccatcg aaaagaccat ctccaaggcc aagggacagc ctcgcgagcc ccaggtgtac   1140
   acactgcctc ccagcaggga cgagctgaca aagaatcagg tgtccctgac ctgtctcgtg   1200
   aaaggcttct accccagcga cattgccgtg gaatgggaga gcaacggcca gcccgagaac   1260
   aactacaaga ccaccccccc tgtgctggac agcgacggct cattcttcct gtactccaag   1320
   ctgaccgtgg acaagtccag gtggcagcag ggcaacgtgt tcagctgctc cgtgatgcac   1380
   gaggccctgc acaaccacta cacccagaag tccctgagcc tgagccctgg caagtgatag   1440
   ggcgcgcc                                                            1448
<210> 18
   <211> 725
   <212> DNA
   <213> Artificial Sequence
<220>
   <223> Séquence nucléotidique codant la chaîne légère telle que synthétisée
<400> 18
   actagtgccg ccaccatgcg atggtcctgg atcttcctgc tgctgctgag catcaccagc 60
   gccaacgccg ccatccagct gacccagagc cctagctctc tgagcgccta cgtgggcgac 120
   aaagtgacca tcacctgtca cgccagccag ggcatcaaca gctggctggc ctggtatcag 180
   cagaagcccg gcaaggcccc caagctgctg atctacaagg ccagcagcct gctgagcggc 240
   gtgcccagca gattcagcgg ctctggctct ggcaccgact acaccctgac catcagctcc 300
   ctgcagagcg aggacttcgc cagctactac tgcctgcagt acgacagcgc ccctctggcc 360
   ttcggccctg gaacaaagct ggacatcaag aggaccgtgg ccgcaccaag tgtctttatc 420
   ttcccaccca gcgacgagca gctgaagtcc ggcacagctt ccgtcgtgtg cctgctgaac 480
   aacttctacc ctagggaagc caaggtgcag tggaaggtgg acaacgccct gcagtccggc 540
   aactcccagg aaagcgtgac cgagcaggac agcaaggact ccacctacag cctgagcagc 600
   accctgacac tgagcaaggc cgactacgag aaacataagg tgtacgcctg cgaagtgacc 660
   caccagggcc tgtctagccc cgtgaccaag agcttcaaca ggggcgagtg ctagtgaact 720
   ctaga 725
<210> 19
   <211> 18
   <212> PRT
   <213> Artificial Sequence
<220>
   <223> peptide signal
<400> 19
   Met Arg Trp Ser Trp Ile Phe Leu Leu Leu Leu Ser Ile Thr Ser Ala
   1 5 10 15
   Asn Ala
<210> 20
   <211> 53
   <212> DNA
   <213> Artificial Sequence
<220>
   <223> séquence codant le peptide signal
<400> 20
   atgcgatggt cctggatctt cctgctgctg ctgagcatca ccagcgccaa cgc 53